# EUROPEAN PATENT SPECIFICATION

(11) **EP 0 531 642 B1**
(45) Date of publication and mention of the grant of the patent: **21.05.1997**
(21) Application number: 92110975.7
(22) Date of filing: 29.06.1992
(51) Int. Cl.: C07H 7/02, C12P 19/02, A61K 31/71

(54) **Antibiotic LL-E19020 Zeta and LL-E 19029 Eta**
Antibiotikum LL-E19020 Zeta und Antibiotikum LL-E19020 Eta
Antibiotiques LL-E19020 zeta et LL-19020 eta

(30) Priority: 09.09.1991 US 756406
(43) Date of publication of application: 17.03.1993
(73) Proprietor: F. HOFFMANN-LA ROCHE AG, 4070 Basel (CH)
(72) Inventor: Carter, Guy Thomas, Suffern, NY 10901 (US); Williams, David R., Stony Point, NY 10980 (US); Korshalla, Joseph D., Pearl River, NY 10965 (US)
(74) Representative: Kellenberger, Marcus, Dr.

(56) References cited:
- US-A- 4 705 688
- US-A- 4 968 493
- JOURNAL OF CHROMATOGRAPHY, vol. 483, 1989, Amsterdam (NL); D.R. WILLIAMS et al. , pp. 381-390

## Description

### BACKGROUND OF THE INVENTION

### 1. FIELD OF THE INVENTION

The invention relates to new antibiotics designated LL-E19020 Zeta and LL-E19020 Eta, to their production by fermentation and to a process for their recovery and purification.

### 2. DESCRIPTION OF THE PRIOR ART

Hochlowski, et al. disclose in J. Antibiotics, 1300-1315 (1988) unphenelfamycin which differs from LL-E19020 Eta and Zeta in the number of sugar units at position 21. Although similar in structure, LL-E19020 Eta and Zeta have far lower MIC values against clostridium. Minimization of clostridium levels in animals are associated with increased growth.

Antibiotics LL-E19020 Alpha and LL-E19020 Beta are disclosed in U.S. Patent 4,705,688, The Journal Of Antibiotics, 41(10), 1511 - 1514 (1988) and The Journal Of Antibiotics, 42(10), 1489 - 1493 (1989). Antibiotic LL-E19020 Alpha has a trisaccharide group attached at C-21a, a phenylacetate group attached at C-23 and has the structure:

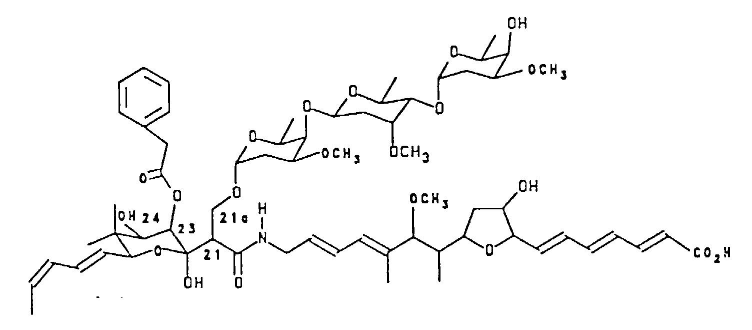

Antibiotic LL-E19020 Beta has a trisaccharide group attached at C-21a, a phenylacetate ester group attached at C-24 and has the structure:

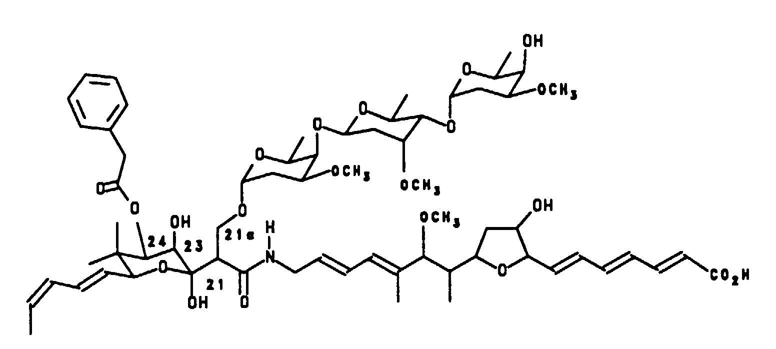

A process for purification of the antibiotic LL-E19020 Alpha by reversed phase HPLC purification is described in J. of Chrom. 484, 381-390(1989). Antibiotics LL-E19020 Alpha and LL-E19020 Beta are also useful for increasing the growth rate of meat producing animals and for treating respiratory disease, fowl cholera and necrotic enteritis as described in U.S. 4,704,276 and U.S. 4,968,493.

A related family of compounds, the phenelfamycins, is reported in The Journal Of Antibiotics, 41(10), 1293 - 1299 (1988); The Journal Of Antibiotics, 41(10), 1300 - 1315 (1988); The Journal Of Antibiotics, 39(10), 1361 - 1367 (1986); The Journal Of Antibiotics, 42(1), 94 - 101 (1989); Antimicrobiol Agents and Chemotherapy, 33(3), 322 - 325 (1989); Program and Abstracts Of The 27th Interscience Conference on Antimicrobial Agents Chemotherapy, No. 995, p 270, New York, October 4 - 7 1987.

### SUMMARY OF THE INVENTION

New antibiotics designated LL-E19020 Zeta and LL-E19020 Eta have now been found. The structure of the new antibiotic LL-E19020 Zeta is:

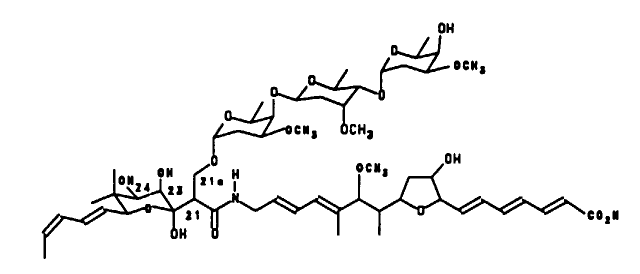

As can be determined from the above structure, antibiotic LL-E19020 Zeta differs from the previously known antibiotics LL-E19020 Alpha and LL-E19020 Beta in that LL-E19020 Zeta lacks the phenyl acetate ester group attached at C-23 or C-24. The physico chemical characteristics of LL-E19020 Zeta are as follows:
1. Molecular weight: 1107 (FABMS=M/Z 1130 corresponding to [M+Na]+)
2. Molecular formula: C₅₇H₈₉NO₂₀
3. Ultraviolet Absorption Spectrum as shown in Figure I.
   UV absorption [MeOH]g ₘₐₓ: 232 nm and 290 nm.
4. IR absorption spectrum as shown in Figure II. IR absorption spectrum [KBr] n ₘₐₓ: 3426, 2973, 2933, 2828, 1700, 1642, 1619, 1447, 1367, 1259, 1191, 1171, 1148, 1097, 1024, 987 cm⁻¹.
5. Proton ¹H NMR[CDCl₃]: Spectrum (300 MHz) as shown in Figure III.
6. Carbon 13 ¹³C NMR[CDCl₃] Spectrum as shown in Figure IV, significant peaks listed below (d from TMS):
   173.5, 170.2, 145.8, 140.3, 136.8, 134.4, 132.1, 130.2, 129.1, 128.5, 128.5, 128.3, 128.3, 127.3, 126.2, 120.7, 100.6, 98.99, 98.53, 97.55, 89.17, 83.23, 81.62, 77.61, 77.20, 76.26, 74.64, 74.38, 74.21, 74.21, 72.51, 70.68, 69.14, 67.53, 66.76, 66.03, 64.60, 56.53, 56.05, 55.70, 55.47, 50.12, 41.69, 39.79, 39.15, 38.81, 32.89, 31.15, 29.88, 23.92, 18.07, 17.25, 16.99, 15.12, 13.48, 10.75, 10.01.
7. High pressure liquid chromatography (HPLC) retention time of 13.2 minutes using a gradient of acetonitrile in aqueous acetic acid.
8. High pressure liquid chromatography (HPLC) retention time of 16.3 minutes using a gradient of dioxane in aqueous acetic acid.

The structure of the new antibiotic LL-E19020 Eta is:

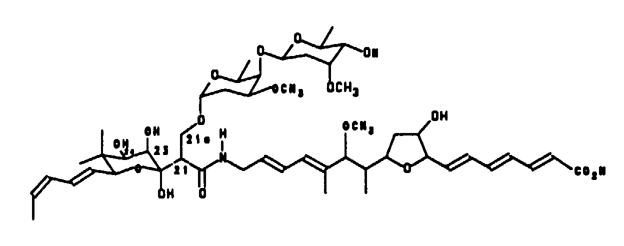

As can be determined from the above structure, antibiotic LL-E19020 Eta differs from the previously known antibiotics LL-E19020 Alpha and LL-E19020 Beta in that LL-E19020 Eta has a disaccharide group rather than a trisaccharide group attached at C-21a and that LL-E19020 Eta lacks the phenyl ester group attached at C-23 or C-24. The physico chemical characteristics of LL-E19020 Eta are as follows:
1. Molecular weight: 963 (FABMS = m/z 986 corresponding to [M + Na]+)
2. Molecular formula: C₅₀H₇₇ NO₁₇ HRFABMS [M + Na]+ = M/Z 986.5075 (calc 986.5089)
3. Ultraviolet Absorption Spectrum as shown in Figure V.
   UV absorption [MeOH]λ ₘₐₓ: 232 nm, and 290 nm.
4. IR absorption spectrum as shown in Figure VI. IR absorption spectrum [KBr]νₘₐₓ: 3433, 3022, 2975, 2934, 2830, 1692, 1641, 1619, 1452, 1380, 1300, 1257, 1186, 1104, 1068, 1023, 1004, 975, 1946cm¹.
5. Proton ¹H NMR[CDCl₃]: Spectrum (300 MHz) as shown in Figure VII.
6. Carbon 13 ¹³C NMR[CDCl₃/CD₃OD]δ Spectrum as shown in Figure VII, significant peaks listed below: ¹³C NMR[CDCl₃]δ from TMS:
   174.0, 169.3, 145.1, 140.0, 136.4, 134.5, 132.0, 130.1, 129.1, 128.8, 128.64, 128.59, 128.0, 127.2, 126.1, 121.2, 99.41, 98.46, 97.22, 89.40, 83.60, 77.68, 76.29, 75.15, 74.81, 74.42, 74.16, 72.44, 70.66, 67.63, 67.19, 66.14, 64.31, 56.34, 55.97, 55.38, 49.85, 41.64, 39.86, 39.12, 38.82, 30.79, 29.59, 23.92, 17.27, 16.78, 15.05, 13.36, 10.70, 9.93.
7. High pressure liquid chromatography (HPLC) retention time of 11.5 minutes using a gradient of acetonitrile in aqueous acetic acid.
8. High pressure liquid chromatography (HPLC) retention time of 14.2 minutes using a gradient of dioxane in aqueous acetic acid.

The new antibiotics LL-E19020 Zeta and LL-E19020 Eta are formed along with LL-E19020 Alpha and LL-E19020 Beta during cultivation under controlled conditions of a strain of Streptomyces lydicus ssp. tanzanius, NRRL 18036. The new antibiotics LL-E19020 Zeta and LL-E19020 Eta are separated from LL-E19020 Alpha and LL-E19020 Beta and subsequently purified by high pressure liquid chromotography (HPLC).

### Brief Description Of The Drawings

Figure I shows the ultraviolet absorption spectrum of LL-E19020 Zeta.

Figure II shows the infrared absorption spectrum of LL-E19020 Zeta.

Figure III shows the proton nuclear magnetic resonance spectrum of LL-E19020 Zeta.

Figure IV shows the carbon-13 nuclear magnetic resonance spectrum of LL-E19020 Zeta.

Figure V shows the ultraviolet absorption spectrum of LL-E19020 Eta.

Figure VI shows the infrared absorption spectrum of LL-E19020 Eta.

Figure VII shows the proton nuclear magnetic resonance spectrum of LL-E19020 Eta.

Figure VIII shows the carbon-13 nuclear magnetic resonance spectrum of LL-E19020 Eta.

### Description Of The Preferred Embodiments

The antibiotics LL-E19020 Zeta and Eta are produced by fermentation of a strain of Streptomyces lydicus, ssp. tanzanius, NRRL 18036, in an aqueous nutrient medium containing assimilable sources of carbon and nitrogen under submerged aerobic conditions. This microorganism is maintained in the culture collection of the Medical Research Division, American Cyanamid Company, Pearl River, New York as culture number LL-E19020. A viable culture of this new micro-organism has been deposited with the Patent Culture Collection Laboratory, Northern Regional Research Center, U.S. Department of Agriculture, Peoria, Illinois 61604, and has been added to its permanent collection. It has been assigned the strain designation NRRL 18036 by said depository.

Culture LL-E19020 produces short spiral spore chains, 10-50 spores long, with occasional longer chains. These tend to coalesce to form dry blackish masses on such ISP media as oatmeal and inorganic salts-starch. The spores have smooth surfaces as assessed by electron microscopy. The strain contains the L isomer of diaminopimelic acid, and may thus be assigned to the genus Streptomyces.

In the ISP tests for utilization of carbohydrates, LL-E19020 shows growth on arabinose, fructose, inositol, mannitol, reffinose, rhamnose, sucrose and xylose. Cellulose is not utilized.

The reactions of LL-E19020 in the Gordon physiological series are compared in the following Table I with those of Streptomyces lydicus ISP 5461 which it most closely resembles morphologically and physiologically.

Because LL-E19020 differs from ISP 5461 in five(5) characteristics (xanthine hydrolysis, decarboxylation of oxalate, acid from erythritol, rhamnose and β-methyl-D-xyloside) it is designated as a subspecies of Streptomyces lydicus.

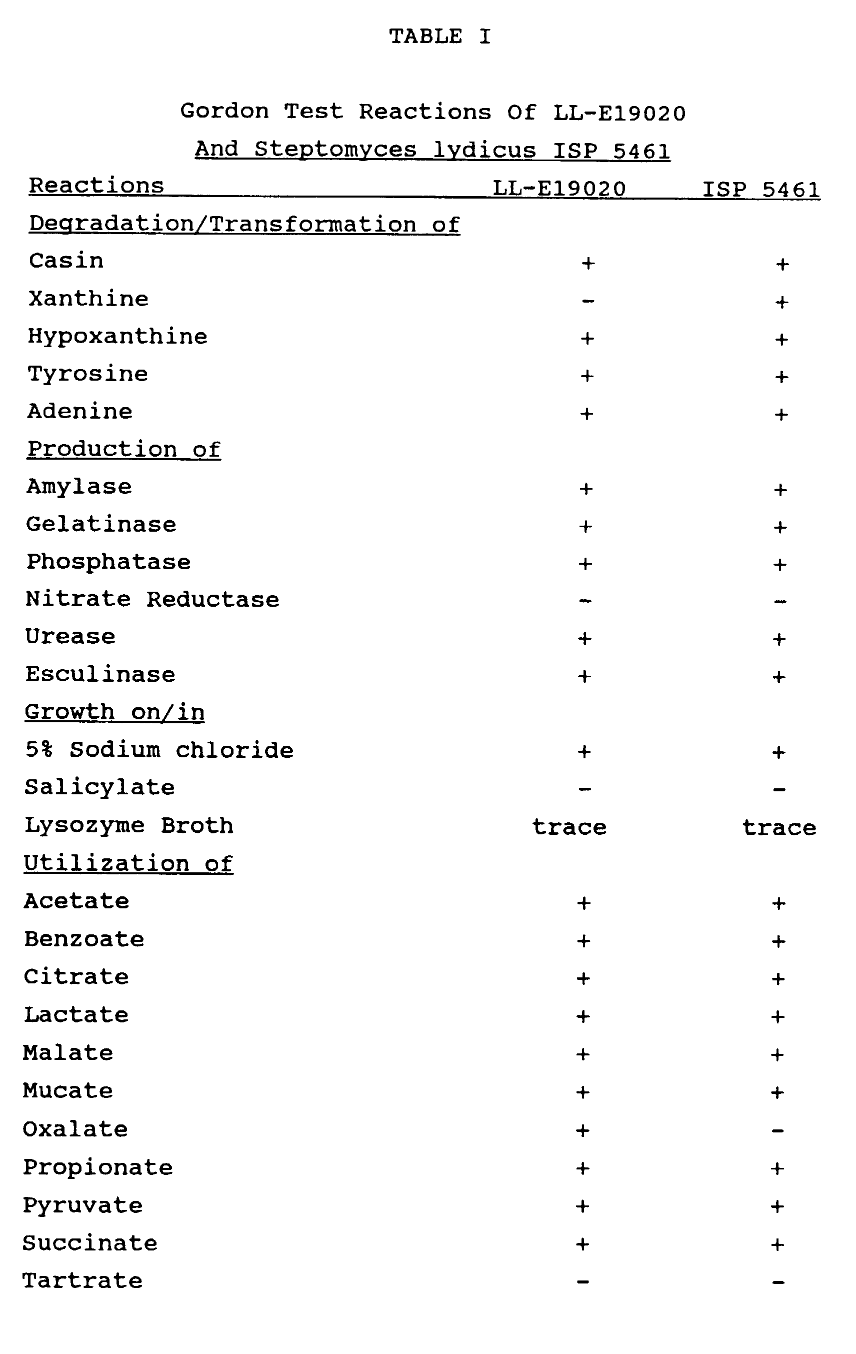

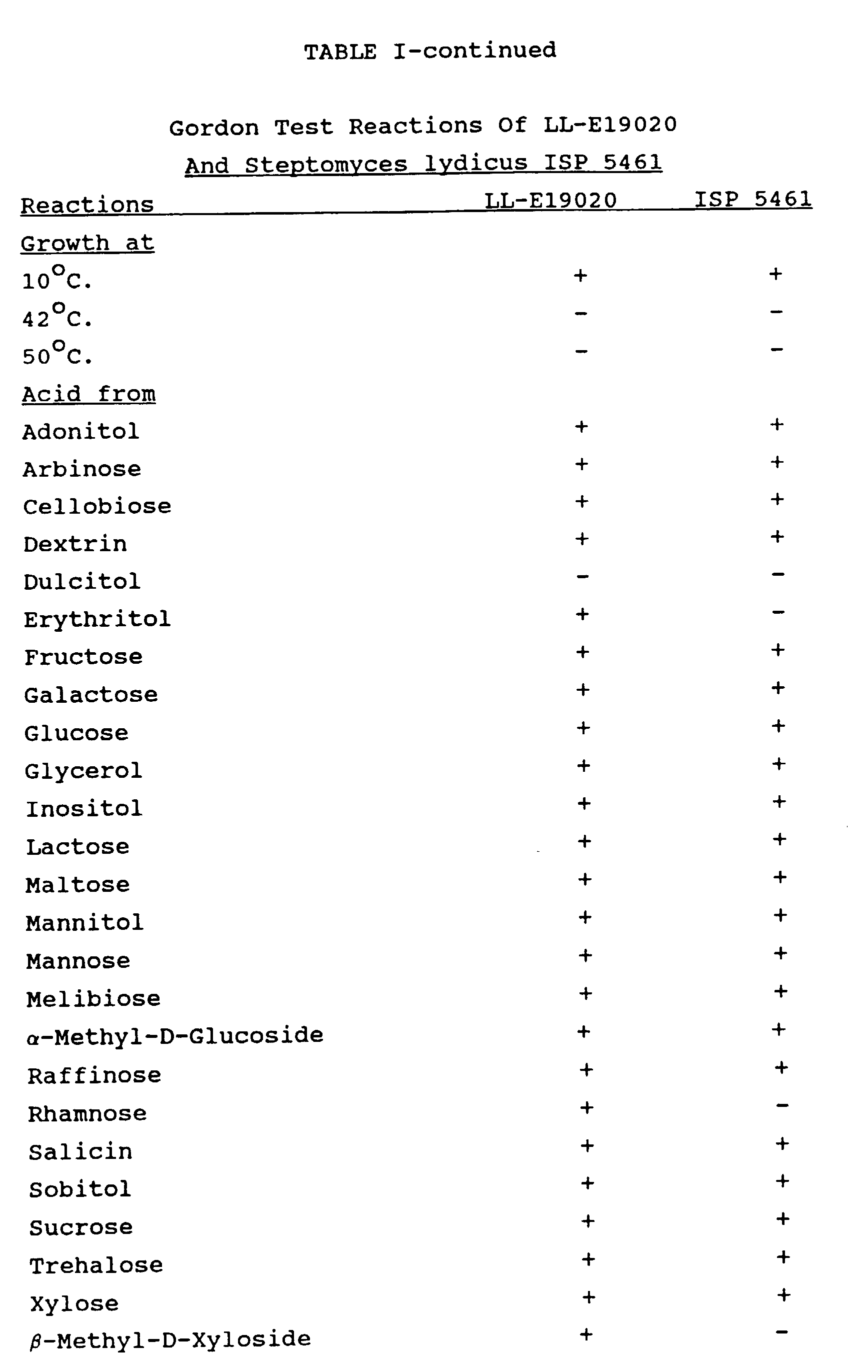

It is to be understood that for the production of these new antibacterial agents the present invention is not limited to this particular organism or to organisms fully answering the above characteristics which are given for illustrative purposes only. In fact, it is desired and intended to include the use of mutants produced from this organism by various means such as exposure of X-radiation, ultraviolet radiation, N'-methyl-N'-nitro-N-nitrosoguanidine, actinophages and the like.

Cultivation of Streptomyces lydicus ssp. tanzanius NRRL 18036 may be carried out in a wide variety of liquid culture media. Media which are useful for the production of LL-E19020 Zeta and LL-E 19020 Eta include an assimilable source of carbon, such as dextrin, sucrose, molasses, glycerol, etc; an assimilable source of nitrogen such as protein, protein hydrolysate, polypeptides, amino acids, corn steep liquor, etc; and inorganic anions and cations, such as potassium, sodium, ammonium, calcium, sulfate, carbonate, phosphate, chloride, etc. Trace elements such as boron, molybdenum, copper, etc., are supplied as impurities of other constituents of the media. Aeration in tanks and bottles is supplied by forcing sterile air through or onto the surface of the fermenting medium. Further agitation in tanks is provided by a mechanical impeller. An antifoam agent such as silicone oil may be added as needed.

The antibiotics LL-E19020 Zeta and LL-E19020 Eta are recovered from the fermentation broth by adsorption on a nonionic adsorption resin.

### EXAMPLE 1

### INOCULUM PREPARATION

A typical medium used to grow the primary inoculum is prepared according to the following formula:

| | |
|---|---|
| Dextrose | 1.0% |
| Dextrin | 2.0% |
| Yeast extract | 0.5% |
| NZ Amine A | 0.5% |
| Calcium carbonate | 0.1% |
| Water qs | 100.0% |
| **NOTE:** NZ Amine A is a pancreatic digest of casein, registered trademark of Scheffield Chemical, Norwich, New York. | |

This medium is sterilized and 100 ml, in a 500 ml flask, is inoculated with Streptomyces lydicus ssp. tanzanius NRRL 18036. The medium is then placed on a rotary shaker and incubated at 28°C for 48 hours providing a primary inoculum. This primary inoculum is them used to inoculate 10 liters of the same sterile medium in a tank except that 0.3% v/v silicone antifoam is also added. This culture is grown for 48 hours providing a secondary inoculum. This secondary inoculum is then used to inoculate 300 liters of the production medium in a fermenter.

### EXAMPLE 2

### FERMENTATION

A fermentation production medium of the following formulation is prepared:

| | |
|---|---|
| Dextrin | 7.0% |
| Dextrose | 0.5% |
| Soy flour | 1.5% |
| Corn steep liquor | 0.5% |
| Calcium carbonate | 0.5% |
| Silicone antifoam | 0.3% |
| Water qs | 100.0% |

This medium is sterilized and is then inoculated with 10 liters of secondary inoculum from Example 1 to a final volume of 300 liters. The fermentation is conducted at 30°C with a sterile air flow of 0.67 liters of air per liter of mash per minute and agitation by an impeller driven at 200 rpm for 92-93 hours, at which time the mash is harvested.

### EXAMPLE 3

### ISOLATION AND PURIFICATION OF LL-E19020 ZETA AND LL-E19020 ETA

The harvest mash from two (2) fermentations conducted as described in Example 2 making a total volume of 503 liters is diluted with 6 liters of toluene. The pH is adjusted to 4.5 using concentrated hydrochloric acid. While stirring, 250 liters of methyl alcohol is added. Stirring is continued over 2 hours and the pH is continuously monitored. To the mixture is added 50 pounds of diatomaceous earth followed by stirring for 15 minutes. The mixture is filtered through a filter press with the press washed with 75 liters of methyl alcohol. The total volume collected is 697 liters. A 45 liter HP-20® column is prepared by washing the resin with 100 liters of deionized water at a rate of 1 to 2 liters/minute followed by 120 liters of 1:1 1N sodium hydroxide/methyl alcohol at a rate of 1 to 2 liters/minute followed by 120 liters of 1N sulfuric acid at a rate of 1 to 2 liters/minute followed by 100 liters of deionized water at a rate of 1 to 2 liters/minute. The pH of the eluate is checked and additional deionized water wash could be needed to bring the pH to between 6 and 7. The column is further washed with 100 liters of methyl alcohol at a rate of 1 to 2 liters/minute followed by 100 liters of deionized water. The column is further washed at a rate of 1 to 2 liters/minute with a solution of 108 liters of acetone and 12 liters of water followed by 100 liters of acetone at a rate of 1 to 2 liters/minute and concluded with 100 liters of deionized water at a rate of 1 to 2 liters/minute. The 697 liters of liquid from the filter press is added to the prepared HP-20® column at a rate of 1 liter/minute. The column is further washed with 120 liters of deionized water at a rate of 1 liter/minute followed by a solution of 64 liters of deionized water and 16 liters of acetone at a rate of 1 liter/minute. Four 20 liter fractions are collected and designated F1-F4. The column is further washed with a solution made from 48 liters of deionized water and 32 liters of acetone at a rate of 0.5 to 1 liter/minute to give four 20 liter fractions which are collected and labeled F5-F8. Further washing of the column with a solution made from 32 liters of deionized water and 48 liters of acetone at a rate of 0.5 to 1 liter/minute affords four 20 liter collected factions designated F9-F12. The column is further washed with a solution made from 16 liters of water and 64 liters of acetone at a rate of 0.5 to 1 liter/minute to afford four 20 liter collected fractions designated as F13-F16. Further washing of the column with acetone at a rate of 0.5 to 1 liter/minute affords four 20 liter fractions designated F17-F20. Fraction 16 is concentrated and freeze dried to afford 36.8 g of material which is purified by high pressure liquid chromatography (HPLC) on a C₁₈ reverse phase column (5.0 x 25cm) by elution with 50-52% dioxane in 1% aqueous acetic acid. Thirteen fractions are collected from the HPLC. Fraction 9 is further purified by high pressure liquid chromatography on a C₁₈ reverse phase column (2.5 x 25cm) by elution with 65% methyl alcohol in 1% acetic acid to afford 16.0mg of LL-E19020 Eta. Fraction 13 is further purified by reverse phase chromatography on a C₁₈ column (2.5 X 25cm) by elution with 67.5% methyl alcohol in 1% acetic acid to afford 29.3mg of LL-E19020 Zeta.

### ANALYTICAL HIGH PRESSURE LIQUID CHROMATOGRAPHY (HPLC)

The LL-E19020 Eta and Zeta components are analyzed using two different analytical HPLC systems. Their retention times compared to LL-E19020 α and β are indicated below:

| | RETENTION TIME (MINUTES) | |
|---|---|---|
| COMPONENTS | SYSTEM A | SYSTEM B |
| LL-E19020α (alpha) | 22.7 | 23.5 |
| LL-E19020β (beta) | 27.6 | 26.7 |
| LL-E19020η (eta) | 11.5 | 14.2 |
| LL-E19020ζ (zeta) | 13.2 | 16.3 |

A. HPLC system: Alltech adsorbosphere HS® 5µ C18 column (4.6X250 mm) with guard column, eluted with a gradient of acetonitrile in 1% aqueous acetic acid. The starting composition is 40% acetonitrile linearly increasing to 70% over 25 minutes and holding at 70% for 5 minutes. The flow rate is 1.0 mL per minute.

B. HPLC system: Alltech adsorbosphere HS® 51 C18 (4.6X250 mm) with guard column, eluted with a gradient of dioxane in 1% aqueous acetic acid. The starting composition is 55% dioxane, increasing to 70% over 25 minutes and holding at 70% for 5 minutes. The flow rate is 1.0 mL per minute.

### EXAMPLE 4

### In Vitro Antibacterial Activity Of LL-E19020 Zeta and LL-E19020 Eta

The in vitro antibacterial activity of LL-E19020 Zeta and LL-E19020 Eta is determined against a spectrum of gram positive and gram negative bacteria by a standard agar dilution method. Mueller-Hinton agar containing 5% sheep blood and two-fold decreasing concentrations of LL-E19020 Zeta and LL-E19020 Eta respectively is poured into petri dishes. The agar surfaces are inoculated with 1 to 5X10⁴ colony forming units of bacteria by means of the Steers replicating device. The lowest concentration of antibiotic that inhibited growth of a bacterial strain after 18 hours incubation is recorded as the minimal inhibitory concentration for that strain.

### Minimum Inhibitory Concentration Procedure By Agar Dilution

1. Serial two-flow dilutions of drug are prepared in Mueller-Hinton broth in a range of 2560 mg/ml-0.15 mg/ml plus a solvent control.
2. Two milliliters of drug dilution (10X) are added to sterile screw cap bottles to which 18 ml of Mueller-Hinton agar containing 5.6% defibrinated sheep blood is added. Final drug concentration ranges 256 mg/ml-0.015 mg/ml in agar containing 5% sheep blood.
3. A few isolated colonies of each test organism are inoculated into 5 ml trypticase soy broth or brain heart infusion broth. The cultures are shaken at 35°C. for 5 hours.
4. Each culture is diluted 1:50 (10^{-1.7}) in Mueller-Hinton broth and applied to agar plates using a Steers replicator. Control plates should be seeded last to ensure that viable organisms were present throughout the procedure. Inoculated agar plates are allowed to stand undisturbed until the inoculum spots are completely absorbed.
5. The plates are inverted and incubated at 35^{o}C. for 18 hours with CO₂.
6. The minimum inhibitory concentration (MIC) is taken as the lowest concentration of antimicrobial agent at which complete inhibition occurs. A very fine, barely visible haze or a single colony is disregarded.

The results are as follows:

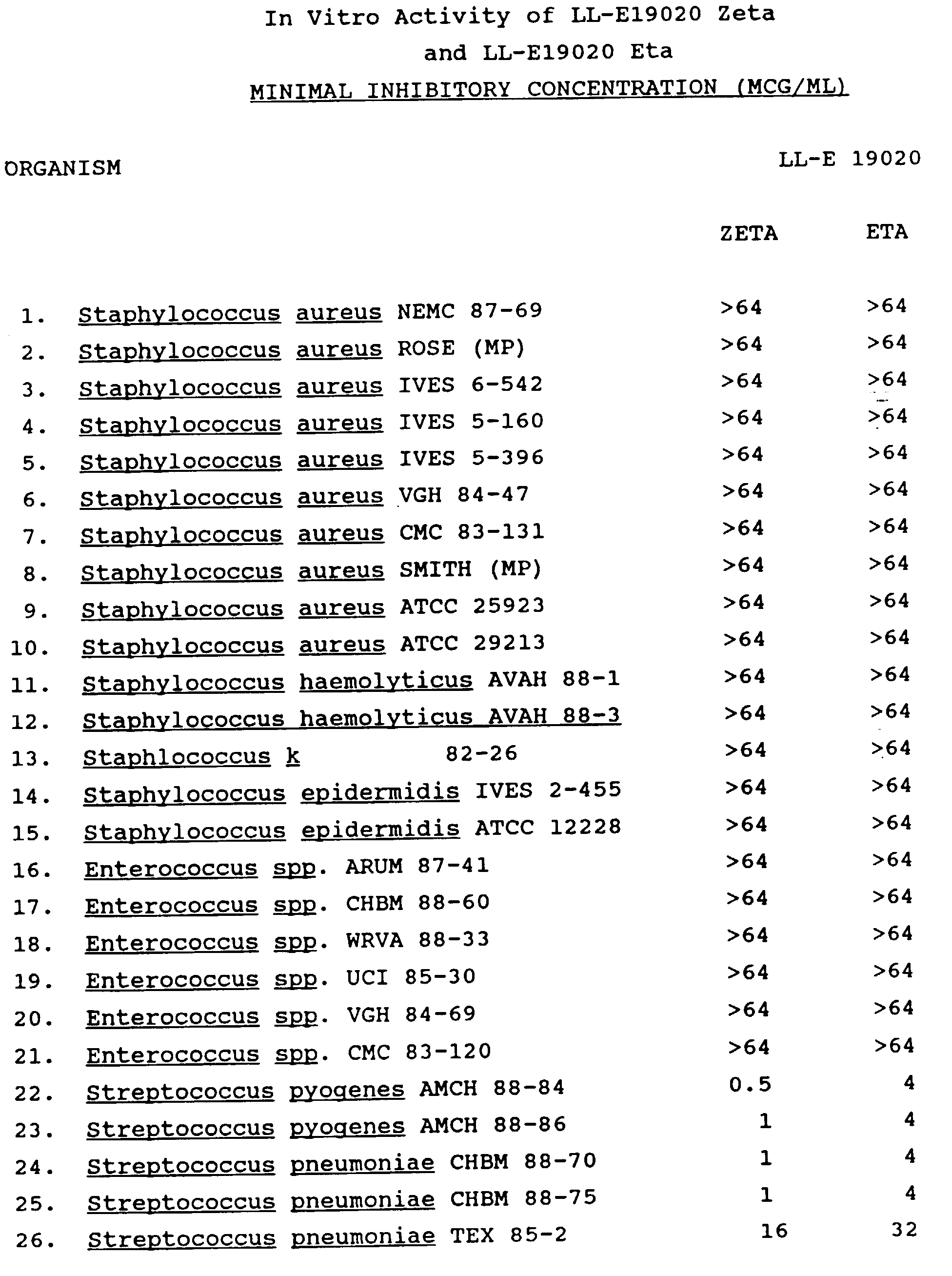

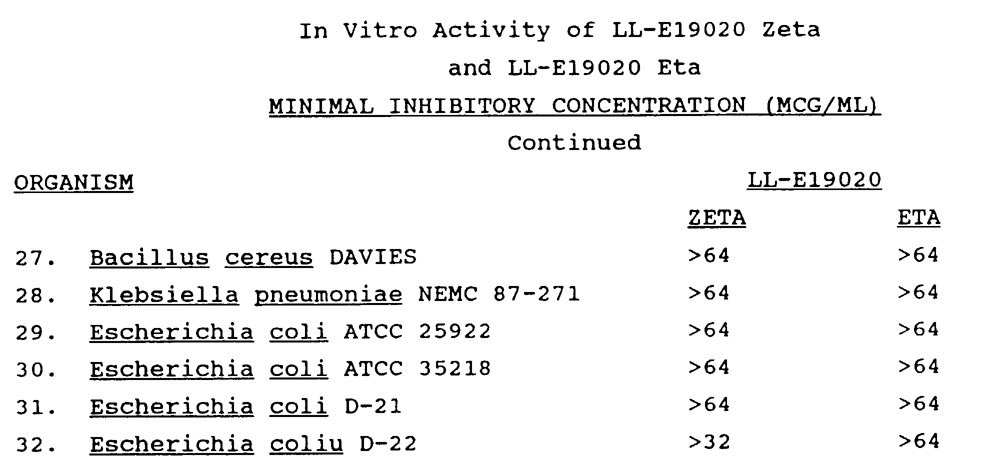

The in vitro antibacterial activity of LL-E19020 Zeta and LL-E19020 Eta is also determined against a spectrum of anaerobic bacteria method.
The results are as follows:

| In vitro Activity of LL-E19020 Zeta and LL-E19020 Eta MINIMAL INHIBITORY CONCENTRATION (MCG/ML) | | |
|---|---|---|
| ORGANISM | LL-E19020 ZETA | LL-E19020 ETA |
| 1. B.fragilis ATCC 25285 | >64 | >64 |
| 2. B.vulgatus ATCC 29327 | 32 | 2 |
| 3. B.theta ATCC 29741 | >64 | >64 |
| 4. B.theta ATCC 29742 | >64 | >64 |
| 5. C.perf. ATCC 13124 | 32 | 4 |
| 6. C.diff. ATCC 17858 | 8 | 4 |
| 7. Ps. mag ATCC 29328 | 0.06 | ≥0.03 |
| 8. Ps. mag ATCC 14956 | ≥0.03 | ≥0.03 |
| 9. Ps. asarc. ATCC 29743 | ≥0.03 | ≥0.03 |

As can be seen from the in vitro data above, LL-E19020 Zeta and LL-E19020 Eta are antibacterial agents.

Antibiotics LL-E19020 Zeta and LL-E19020 Eta derive their utility from antibacterial activity. For example these antibiotics may be used in the suppression of bacterial infections, as a topical antibacterial agent and as a general disinfectant for laboratories. In addition to their antibacterial activity these compounds are effective as an anticoccidial agent in poultry and as a growth promotant and anthelmintic agent.

In therapeutic use, the compounds of this invention may be administered in the form of conventional pharmaceutical compositions appropriate for the intended use. Such compositions may be formulated so as to be suitable for oral, parenteral, or topical administration. The active ingredient may be combined in admixture with a nontoxic pharmaceutically acceptable carrier, which carrier may take a wide variety of forms depending on the form of preparation desired for administration, ie, oral, parenteral or topical.

When the compounds are employed for the above utility, they can be combined with one or more pharmaceutically acceptable carriers, for example, solvents, diluents and the like, and may be administered orally in such forms as tablets, capsules, dispersible powders, granules, or suspensions containing, for example, from about 0.05 to 5% of suspending agent, syrups containing, for example from about 10 to 50% of sugar, and elixirs containing, for example, from about 20 to 50% ethanol, and the like, or parenterally in the form of sterile injectable solutions or suspensions containing from about 0.05 to 5% suspending agent in an isotonic medium. Such pharmaceutical preparations may contain, for example, from about 0.05 up to about 90% of the active ingredient in combination with the carrier, more usually between about 5% and 60% by weight.

An effective amount of compound from 0.2 mg/kg of body weight to 100.0 mg/kg of body weight should be administered one to five times per day via any topical route of administration including but not limited to oral, parenteral (including subcutaneous, intravenous, intramuscular, intrasternal injection or infusion techniques), by inhalation spray, or rectally, in dosage unit formulations containing conventional non-toxic pharmaceutically acceptable carriers, adjuvants and vehicles. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

These active compounds may be administered orally as well as by intravenous, intramuscular, or subcutaneous routes. Solid carriers include starch, lactose, dicalcium phosphate, microcrystalline cellulose, sucrose and kaolin, while liquid carriers include sterile water, polyethylene glycols, non-ionic surfactants and edible oils such as corn, peanut and sesame oils, as are appropriate to the nature of the active ingredient and the particular form of administration desired. Adjuvants customarily employed in the preparation of pharmaceutical compositions may be advantageously included, such as flavoring agents, coloring agents, preserving agents, and antioxidants, for example, vitamin E, ascorbic acid, BHT and BHA.

The preferred pharmaceutical compositions from the stand-point of ease of preparation and administration are solid compositions, particularly tablets and hard-filled or liquid-filled capsules. Oral administration of the compound is preferred.

These active compounds may also be administered parenterally or intraperitoneally. Solutions or suspensions of these active compounds as a free base or pharmacologically acceptable salt can be prepared in water suitably mixed with a surfactant such as hydroxy propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oil.

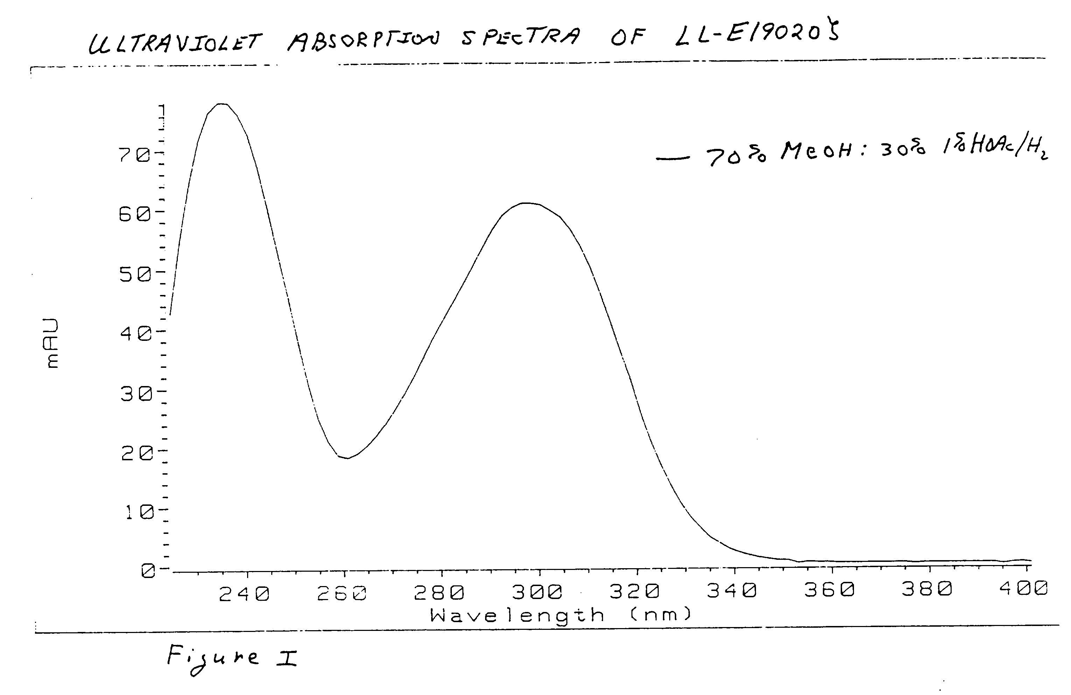

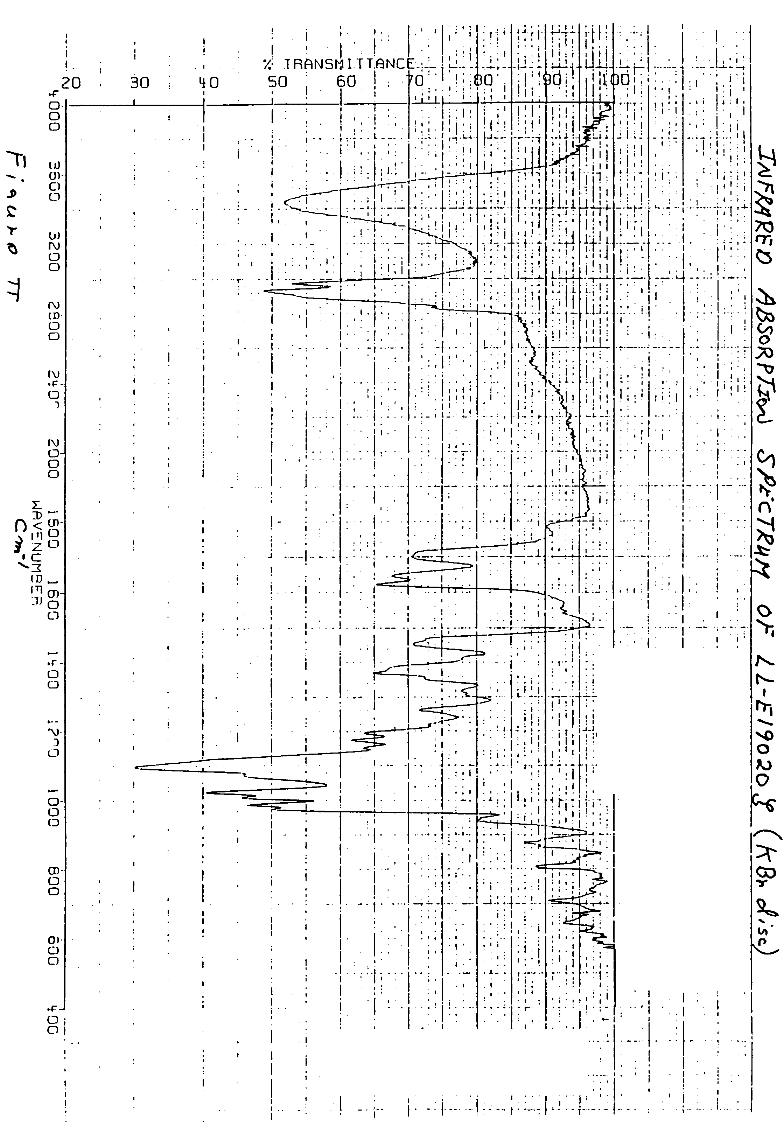

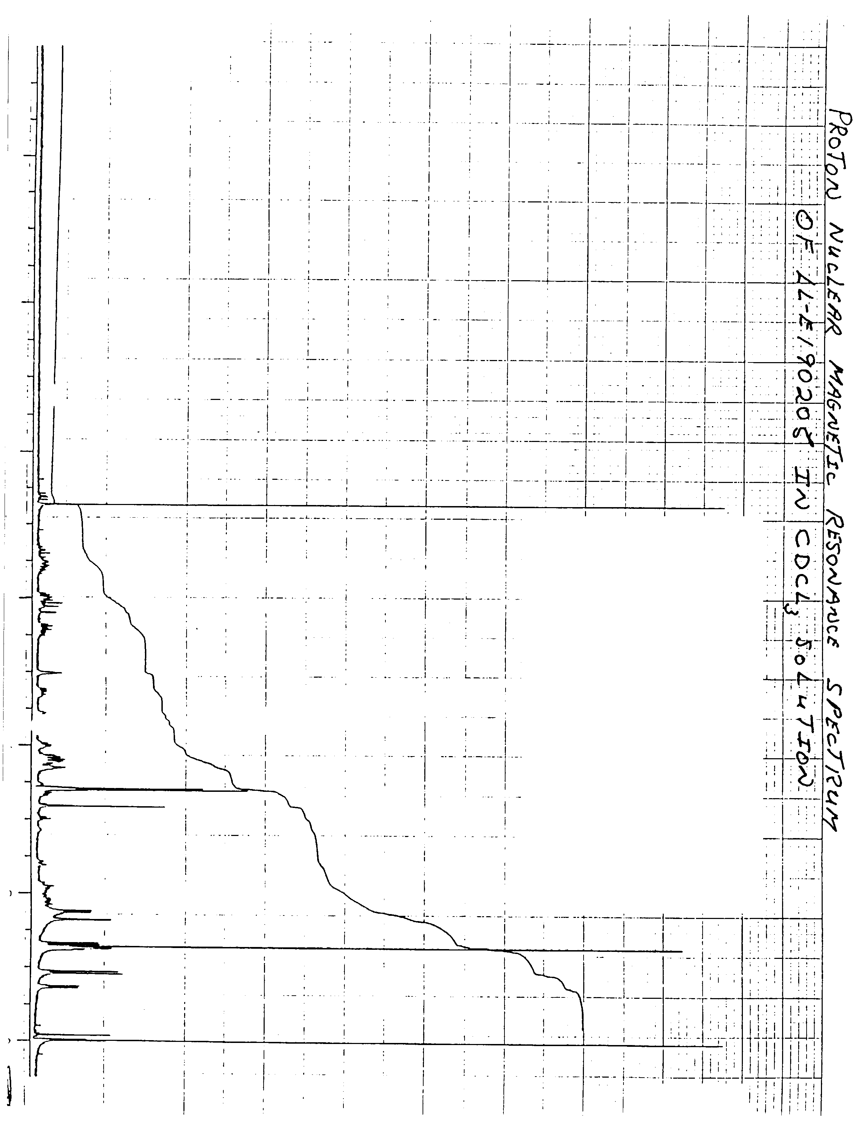

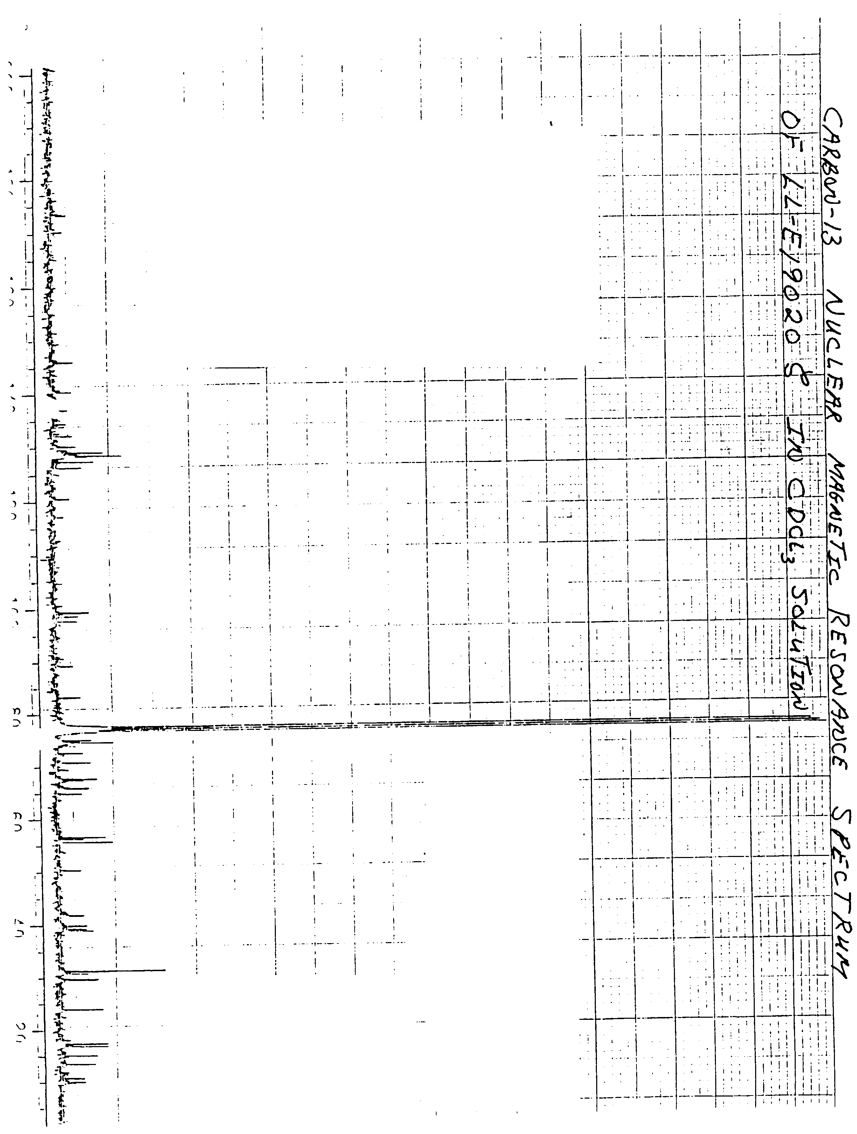

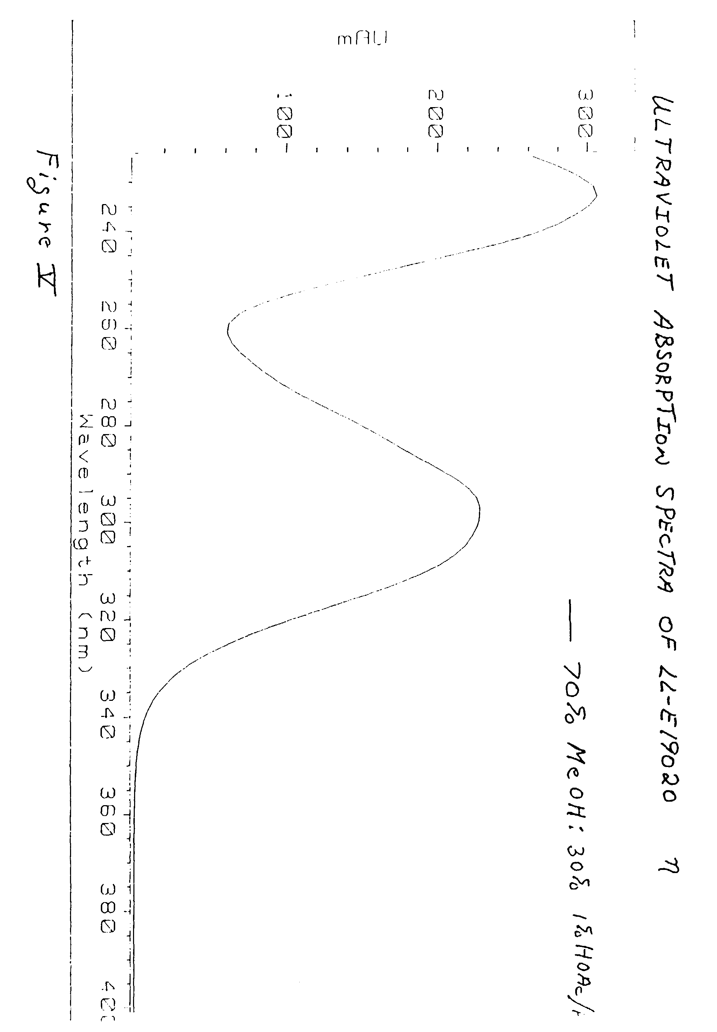

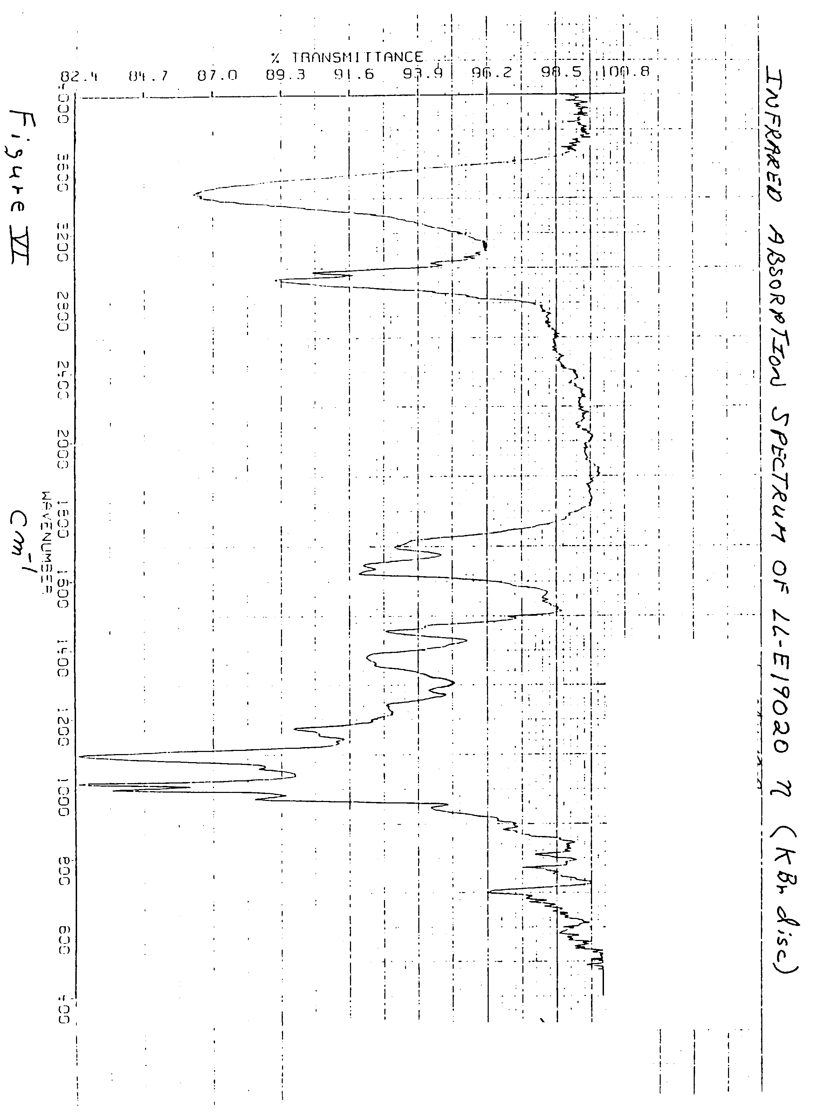

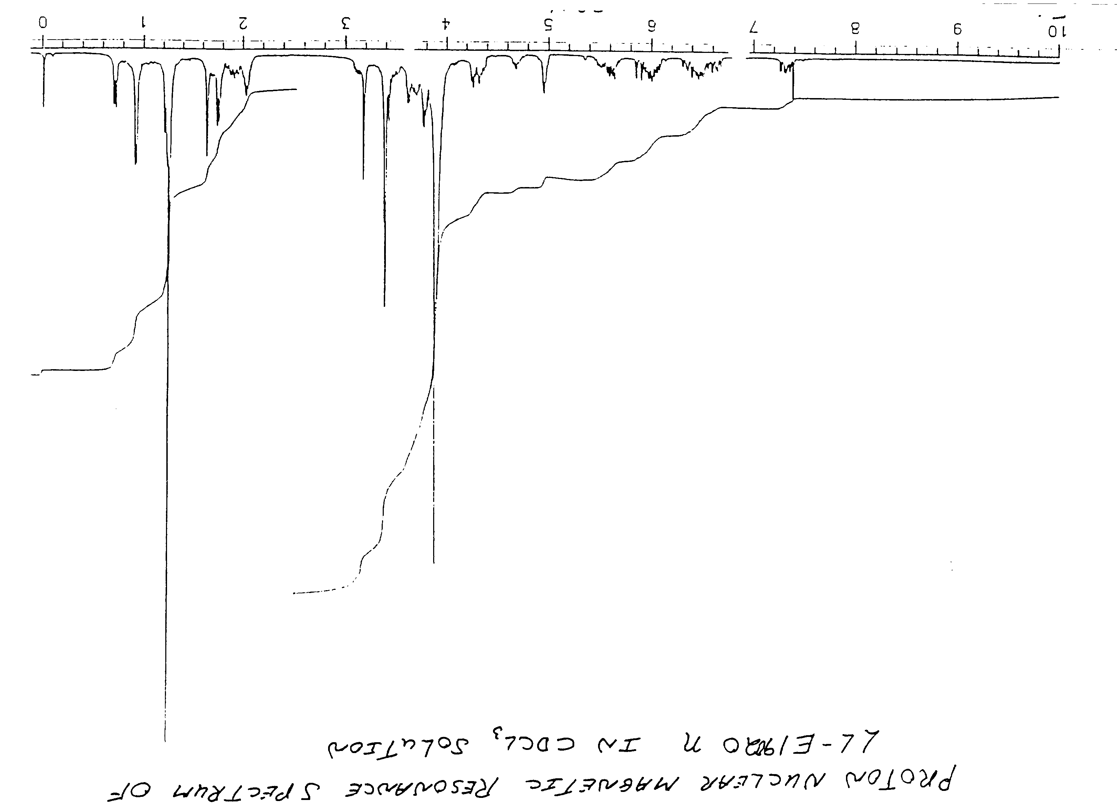

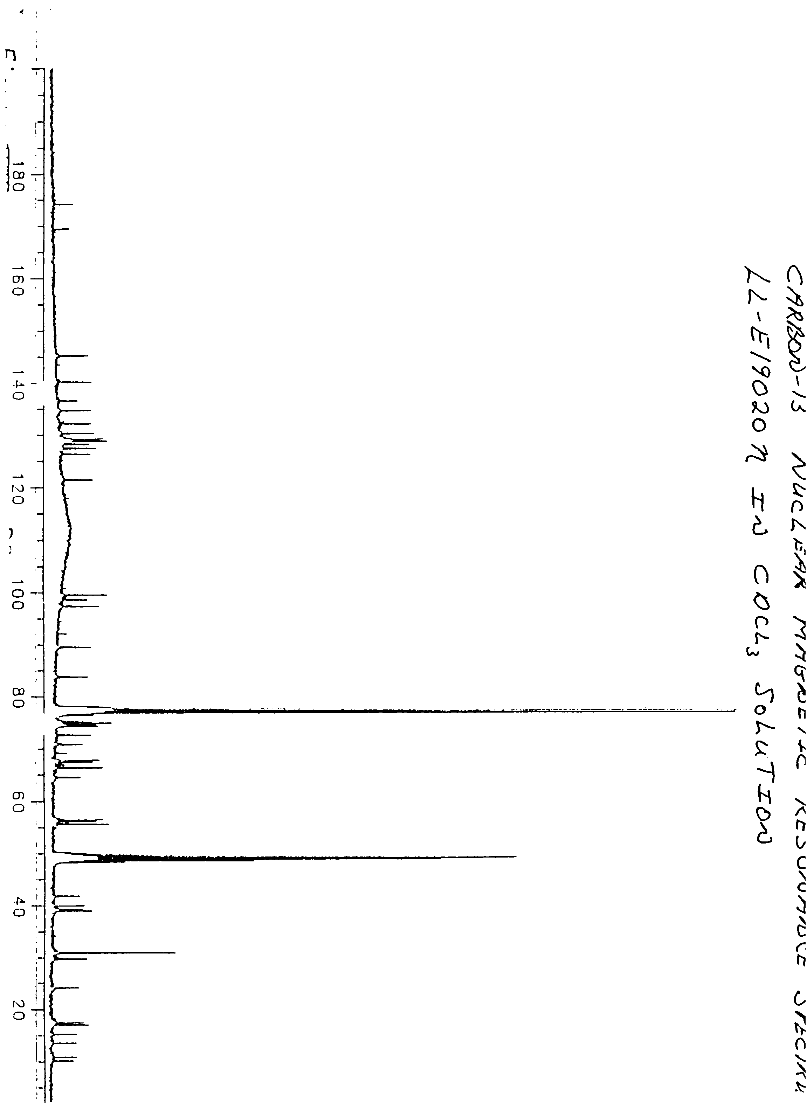

## Claims (Claims for the following Contracting State(s): AT, BE, CH, LI, DE, DK, FR, GB, IT, LU, NL, PT, SE)

1. A compound LL-E19020 Zeta comprising
(a) the structure 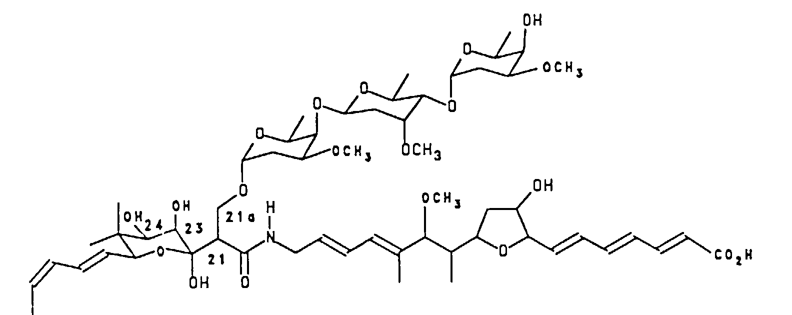
(b) a molecular weight of 1107 (FABMS = M/Z 1130 corresponding to [M+Na]+);
(c) a characteristic ultraviolet absorption spectrum as shown in Figure I of the attached drawings;
(d) a characteristic infrared absorption spectrum as shown in Figure II of the attached drawings;
(e) a characteristic proton nuclear magnetic resonance spectrum as shown in Figure III of the attached drawings;
(f) a characteristic carbon-13 nuclear magnetic resonance spectrum as shown in Figure IV of the attached drawings.
(g) a characteristic HPLC retention time of 13.2 minutes using a gradient of acetonitrile in aqueous acetic acid; and
(h) a characteristic HPLC retention time of 16.3 minutes using a gradient of dioxane in aqueous acetic acid.

2. A process for producing antibiotic LL-E 19020 Zeta as defined in Claim 1 which comprises aerobically fermenting the organism Streptomyces lydicus ssp. tanzanius NRRL 18036 or mutants thereof in a liquid medium containing assimilable sources of carbon, nitrogen and inorganic salts, until substantial antibiotic activity is imparted to said medium and then recovering the antibiotic LL-E19020 Zeta therefrom.

3. A process for producing antibiotic LL-E 19020 Zeta as defined in Claim 1 which comprises aerobically fermenting a liquid medium containing assimilable sources of carbon, nitrogen and inorganic salts, which medium has been inoculated with a viable culture of the organism Streptomyces lydicus ssp. tanzanius NRRL 18036 or mutants thereof, maintaining said fermentation culture at a temperature of 25-32°C for a period of about 80-200 hours, harvesting the mash and extracting the antibiotic.

4. Use of the antibiotic LL-E19020 Zeta as defined in Claim 1 for the preparation of a medicine for treating bacterial infections in warm-blooded animals.

5. A antibiotic pharmaceutical composition which comprises an antibiotic amount of LL-E19020 Zeta as defined in Claim 1 in association with a pharmaceutically acceptable carrier.

6. A compound LL-E19020 Eta comprising
(a) the structure 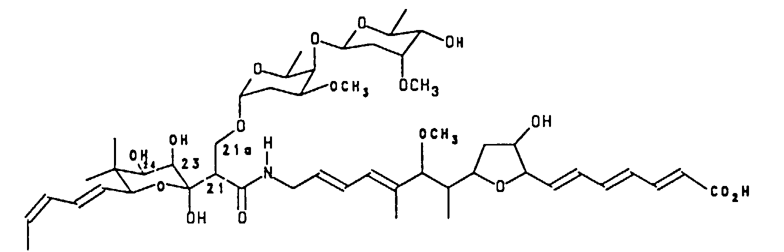
(b) a molecular weight of 963 (FABMS = M/Z 986 corresponding to [M+Na]+).
(c) a characteristic ultraviolet absorption spectrum as shown in Figure V of the attached drawings;
(d) a characteristic infrared absorption spectrum as shown in Figure VI of the attached drawings;
(e) a characteristic proton nuclear magnetic resonance spectrum as shown in Figure VII of the attached drawings;
(f) a characteristic carbon-13 nuclear magnetic resonance spectrum as shown in Figure VIII of the attached drawings;
(g) a characteristic HPLC retention time of 11.5 minutes using a gradient of acetonitrile in aqueous acetic acid; and
(h) a characteristic HPLC retention time of 14.2 minutes using a gradient of dioxane in aqueous acetic acid.

7. A process for producing antibiotic LL-E 19020 Eta as defined in Claim 6 which comprises aerobically fermenting the organism Streptomyces lydicus ssp. tanzanius NRRL 18036 or mutants thereof in a liquid medium containing assimilable sources of carbon, nitrogen and inorganic salts, until substantial antibiotic activity is imparted to said medium and then recovering the antibiotic LL-E19020 Eta therefrom.

8. A process for producing antibiotic LL-E19020 Eta as defined in Claim 6 which comprises aerobically fermenting a liquid medium containing assimilable sources of carbon, nitrogen and inorganic salts, which medium has been inoculated with a viable culture of the organism Streptomyces lydicus ssp. tanzanius NRRL 18036 or mutants thereof, maintaining said fermentation culture at a temperature of 25-32°C for a period of about 80-200 hours, harvesting the mash and extracting the antibiotic.

9. Use of the antibiotic LL-E19020 Eta as defined in Claim 6 for the preparation of a medicine for treating bacterial infections in warm-blooded animals.

10. A antibiotic pharmaceutical composition which comprises an antibiotic amount of LL-E19020 Eta as defined in Claim 6 in association with a pharmaceutically acceptable carrier.

## Claims (Claims for the following Contracting State(s): ES, GR)

1. A process for producing antibiotic LL-E19020 Zeta comprising
(a) the structure 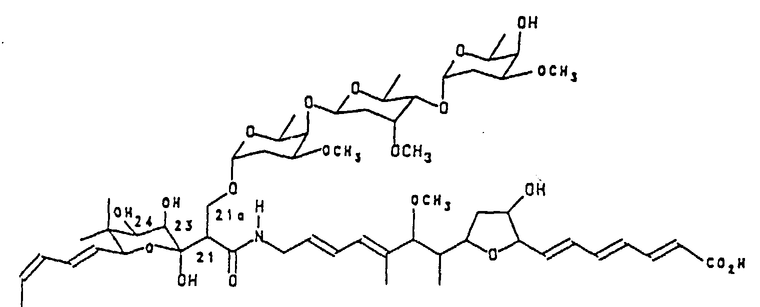
(b) a molecular weight of 1107 (FABMS = M/Z 1130 corresponding to [M+Na]+);
(c) a characteristic ultraviolet absorption spectrum as shown in Figure I of the attached drawings;
(d) a characteristic infrared absorption spectrum as shown in Figure II of the attached drawings;
(e) a characteristic proton nuclear magnetic resonance spectrum as shown in Figure III of the attached drawings;
(f) a characteristic carbon-13 nuclear magnetic resonance spectrum as shown in Figure IV of the attached drawings;
(g) a characteristic HPLC retention time of 13.2 minutes using a gradient of acetonitrile in aqueous acetic acid; and
(h) a characteristic HPLC retention time of 16.3 minutes using a gradient of dioxane in aqeuous acetic acid,
which comprises aerobically fermenting the organism Streptomyces lydicus spp. tanzanius NRRL 18036 or mutants thereof in a liquid medium containing assimilable sources of carbon, nitrogen and inorganic salts, until substantial antibiotic activity is imparted to said medium and then recovering the antibiotic LL-E19020 Zeta therefrom.

2. A process for producing ant ibiotic LL-E19020 Zeta comprising
(a) the structure 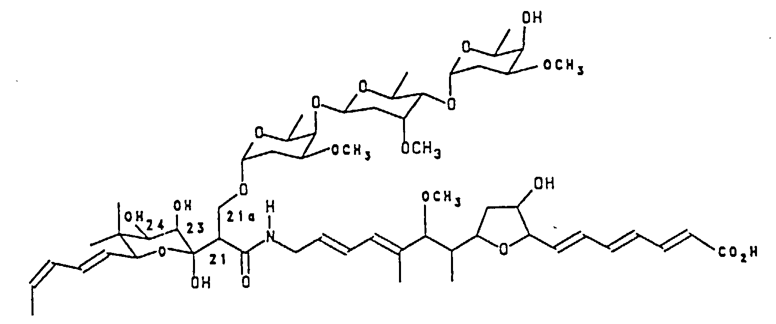
(b) a molecular weight of 1107 (FABMS = M/Z 1130 corresponding to [M+Na]+);
(c) a characteristic ultraviolet absorption spectrum as shown in Figure I of the attached drawings;
(d) a characteristic infrared absorption spectrum as shown in Figure II of the attached drawings;
(e) a characteristic proton nuclear magnetic resonance spectrum as shown in Figure III of the attached drawings;
(f) a characteristic carbon-13 nuclear magnetic resonance spectrum as shown in Figure IV of the attached drawings;
(g) a characteristic HPLC retention time of 13.2 minutes using a gradient of acetonitrile in aqueous acetic acid; and
(h) a characteristic HPLC retention time of 16.3 minutes using a gradient of dioxane in aqeuous acetic acid,
which comprises aerobically fermenting a liquid medium containing assimilable sources of carbon, nitrogen and inorganic salts, which medium has been inoculated with a viable culture of the organism Streptomyces lydicus ssp. tanzanius NRRL 18036 or mutants thereof, maintaining said fermentation culture at a temperature of 25-32°C for a period of about 80-200 hours, harvesting the mash and extracting the antibiotic.

3. Use of the antibiotic LL-E19020 Zeta obtainable according to claim 1 or 2 for the preparation of a medicine for treating bacterial infections in warm-blooded animals.

4. A process for producing an antibiotic pharmaceutical composition which comprises formulating an antibiotic amount of LL-E19020 Zeta obtainable according to claim 1 or 2 with a pharmaceutically acceptable carrier.

5. A process for producing antibiotic LL-E19020 Eta comprising
(a) the structure 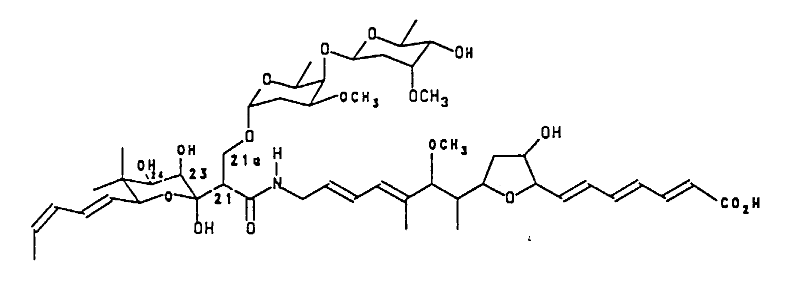
(b) a molecular weight of 963 (FABMS = M/Z 986 corresponding to [M+Na]+);
(c) a characteristic ultraviolet absorption spectrum as shown in Figure V of the attached drawings;
(d) a characteristic infrared absorption spectrum as shown in Figure VI of the attached drawings;
(e) a characteristic proton nuclear magnetic resonance spectrum as shown in Figure VII of the attached drawings;
(f) a characteristic carbon-13 nuclear magnetic resonance spectrum as shown in Figure VIII of the attached drawings;
(g) a characteristic HPLC retention time of 11.5 minutes using a gradient of acetonitrile in aqueous acetic acid; and
(h) a characteristic HPLC retention time of 14.2 minutes using a gradient of dioxane in aqueous acetic acid,
which comprises aerobically fermenting the organism Streptomyces lydicus ssp. tanzanius NRRL 18036 or mutants thereof in a liquid medium containing assimilable sources of carbon, nitrogen and inorganic salts, until substantial antibiotic activity is imparted to said medium and then recovering the antibiotic LL-E19020 Eta therefrom.

6. A process for producing antibiotic LL-E19020 Eta, comprising
(a) the structure 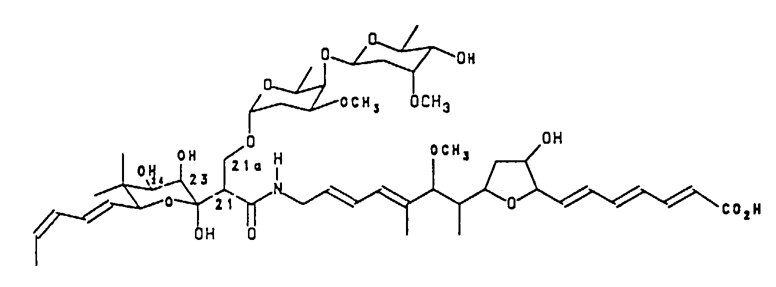
(b) a molecular weight of 963 (FABMS = M/Z 986 corresponding to [M+Na]+);
(c) a characteristic ultraviolet absorption spectrum as shown in Figure V of the attached drawings;
(d) a characteristic infrared absorption spectrum as shown in Figure VI of the attached drawings;
(e) a characteristic proton nuclear magnetic resonance spectrum as shown in Figure VII of the attached drawings;
(f) a characteristic carbon-13 nuclear magnetic resonance spectrum as shown in Figure VIII of the attached drawings;
(g) a characteristic HPLC retention time of 11.5 minutes using a gradient of acetonitrile in aqueous acetic acid; and
(h) a characteristic HPLC retention time of 14.2 minutes using a gradient of dioxane in aqeuous acetic acid,
which comprises aerobically fermenting a liquid medium containing assimilable sources of carbon, nitrogen and inorganic salts, which medium has been inoculated with a viable culture of the organism Streptomyces lydicus ssp. tanzanius NRRL 18036 or mutants thereof, maintaining said fermentation culture at a temperature of 25-32°C for a period of about 80-200 hours, harvesting the mash and extracting the antibiotic.

7. Use of the antibiotic LL-E19020 Eta obtainable according to claim 5 or 6 for preparing a medicine for treating bacterial infections in warm blooded animals.

8. A process for producing an antibiotic pharmaceutical composition which comprises formulating an antibiotic amount of LL-E19020 Eta obtainable according to claim 5 or 6 with a pharmaceutically acceptable carrier.

## Patentansprüche (Patentansprüche für folgende(n) Vertragsstaat(en): AT, BE, CH, LI, DE, DK, FR, GB, IT, LU, NL, PT, SE)

1. Verbindung LL-E19020 Zeta, dadurch gekennzeichnet, dass sie
(a) die Struktur 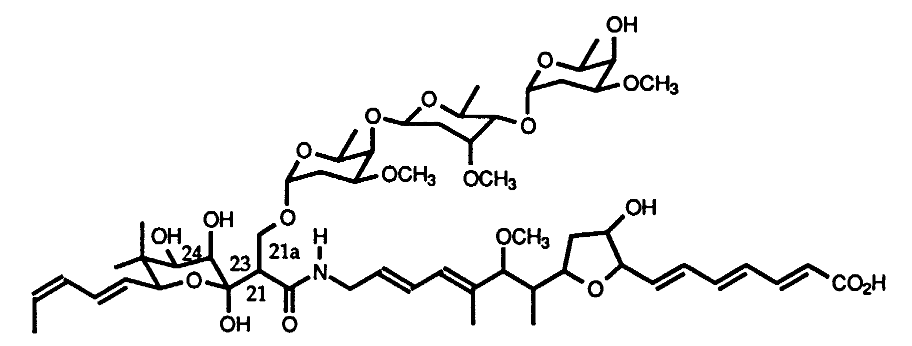
(b) ein Molekulargewicht von 1107 (FABMS=M/Z 1130 entsprechend [M+Na]+);
(c) ein charakteristisches Ultraviolett-Absorptionsspektrum wie in Figur I der beiliegenden Zeichnungen;
(d) ein charakteristisches Infrarot-Absorptionsspektrum wie in Figur II der beiliegenden Zeichnungen;
(e) ein charakteristisches Protonen-NMR-Spektrum wie in Figur III der beiliegenden Zeichnungen;
(f) ein charakteristisches Kohlenstoff-13-NMR-Spektrum wie in Figur IV der beiliegenden Zeichnungen;
(g) eine charakteristische HPLC-Retentionszeit von 13.2 Minuten unter Verwendung eines Eluierungsmittelgradienten von Acetonitril in wässriger Essigsäure; und
(h) eine charakteristische HPLC-Retentionszeit von 16.3 Minuten unter Verwendung eines Eluierungsmittelgradienten von Dioxan in wässriger Essigsäure
aufweist.

2. Verfahren zur Herstellung des in Anspruch 1 definierten Antibiotikums LL-E19020 Zeta, dadurch gekennzeichnet, dass man den Organismus Streptomyces lydicus ssp. tanzanius NRRL 18036 oder einen Mutanten davon in einem flüssigen Medium, welches assimilierbare Quellen von Kohlenstoff, Stickstoff und anorganischen Salzen enthält, aerob fermentiert bis genanntes Medium eine substantielle antibiotische Aktivität aufweist, und danach das Antibiotikum LL-E19020 Zeta daraus isoliert.

3. Verfahren zur Herstellung des in Anspruch 1 definierten Antibiotikums LL-E19020 Zeta, dadurch gekennzeichnet, dass man ein flüssiges Medium, welches assimilierbare Quellen von Kohlenstoff, Stickstoff und anorganischen Salzen enthält und welches mit einer lebensfähigen Kultur des Organismus Streptomyces lydicus ssp. tanzanius NRRL 18036 oder eines Mutanten davon inokuliert wurde, aerob fermentiert, wobei die Fermentationskultur während etwa 80-200 Stunden bei einer Temperatur von 25-32°C gehalten wird, die Maische abtrennt und das Antibiotikum extrahiert.

4. Verwendung des in Anspruch 1 definierten Antibiotikums LL-E19020 Zeta zur Herstellung eines Medikamentes zur Behandlung bakterieller Infektionen in Warmblütern.

5. Eine antibiotische, pharmazeutische Komposition, dadurch gekennzeichnet, dass sie eine antibiotische Menge des in Anspruch 1 definierten LL-E19020 Zeta und einen pharmazeutisch verwendbaren Träger enthält.

6. Verbindung LL-E19020 Eta, dadurch gekennzeichnet, dass sie
(a) die Struktur 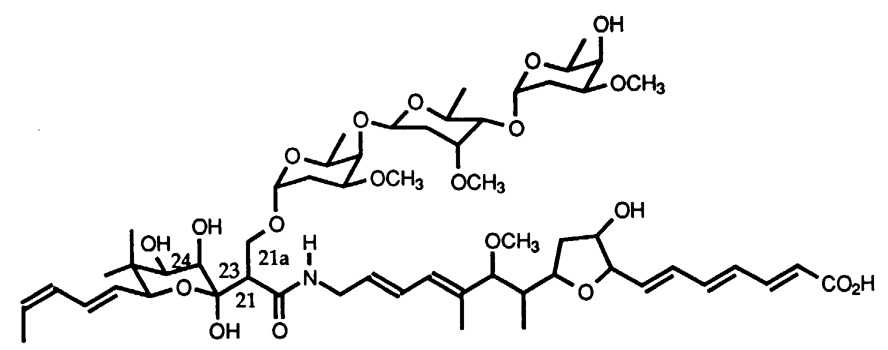
(b) ein Molekulargewicht von 963 (FABMS=M/Z 986 entsprechend [M+Na]+);
(c) ein charakteristisches UItraviolett-Absorptionsspektrum wie in Figur V der beiliegenden Zeichnungen;
(d) ein charakteristisches Infrarot-Absorptionsspektrum wie in Figur VI der beiliegenden Zeichnungen;
(e) ein charakteristisches Protonen-NMR-Spektrum wie in Figur VII der beiliegenden Zeichnungen;
(f) ein charakteristisches Kohlenstoff-13-NMR-Spektrum wie in Figur VIII der beiliegenden Zeichnungen;
(g) eine charakteristische HPLC-Retentionszeit von 11.5 Minuten unter Verwendung eines Eluierungsmittelgradienten von Acetonitril in wässriger Essigsäure; und
(h) eine charakteristische HPLC-Retentionszeit von 14.2 Minuten unter Verwendung eines Eluierungsmittelgradienten von Dioxan in wässriger Essigsäure
aufweist.

7. Verfahren zur Herstellung des in Anspruch 6 definierten Antibiotikums LL-E19020 Eta, dadurch gekennzeichnet, dass man den Organismus Streptomyces lydicus ssp. tanzanius NRRL 18036 oder einen Mutanten davon in einem flüssigen Medium, welches assimilierbare Quellen von Kohlenstoff, Stickstoff und anorganischen Salzen enthält, aerob fermentiert bis genanntes Medium eine substantielle antibiotische Aktivität aufweist, und danach das Antibiotikum LL-E19020 Eta daraus isoliert.

8. Verfahren zur Herstellung des in Anspruch 6 definierten Antibiotikums LL-E19020 Eta, dadurch gekennzeichnet, dass man ein flüssiges Medium, welches assimilierbare Quellen von Kohlenstoff, Stickstoff und anorganischen Salzen enthält und welches mit einer lebensfähigen Kultur des Organismus Streptomyces lydicus ssp. tanzanius NRRL 18036 oder eines Mutanten davon inokuliert wurde, aerob fermentiert, wobei die Fermentationskultur während etwa 80-200 Stunden bei einer Temperatur von 25-32°C gehalten wird, die Maische abtrennt und das Antibiotikum extrahiert.

9. Verwendung des in Anspruch 6 definierten Antibiotikums LL-E19020 Eta zur Herstellung eines Medikamentes zur Behandlung bakterieller Infektionen in Warmblütern

10. Eine antibiotische, pharmazeutische Komposition, dadurch gekennzeichnet, dass sie eine antibiotische Menge des in Anspruch 6 definierten LL-E19020 Eta und einen pharmazeutisch verwendbaren Träger enthält.

## Patentansprüche (Patentansprüche für folgende(n) Vertragsstaat(en): ES, GR)

1. Verfahren zur Herstellung des Antibiotikums LL-E19020 Zeta, welches
(a) die Struktur 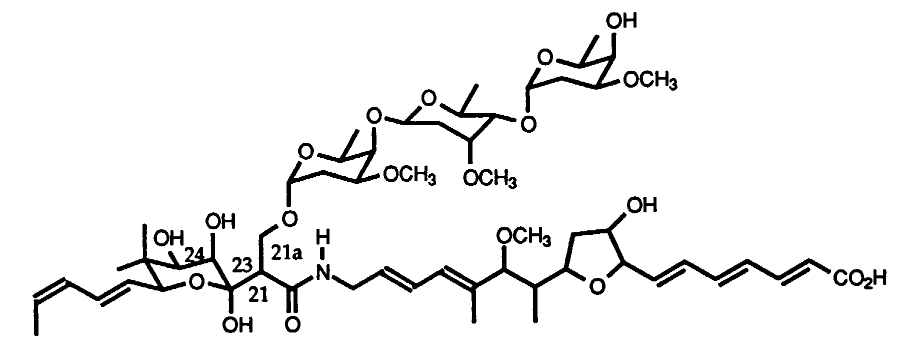
(b) ein Molekulargewicht von 1107 (FABMS=M/Z 1130 entsprechend [M+Na]+);
(c) ein charakteristisches Ultraviolett-Absorptionsspektrum wie in Figur I der beiliegenden Zeichnungen;
(d) ein charakteristisches Infrarot-Absorptionsspektrum wie in Figur II der beiliegenden Zeichnungen;
(e) ein charakteristisches Protonen-NMR-Spektrum wie in Figur III der beiliegenden Zeichnungen;
(f) ein charakteristisches Kohlenstoff-13-NMR-Spektrum wie in Figur IV der beiliegenden Zeichnungen;
(g) eine charakteristische HPLC-Retentionszeit von 13.2 Minuten unter Verwendung eines Eluierungsmittelgradienten von Acetonitril in wässriger Essigsäure; und
(h) eine charakteristische HPLC-Retentionszeit von 16.3 Minuten unter Verwendung eines Eluierungsmittelgradienten von Dioxan in wässriger Essigsäure
aufweist, dadurch gekennzeichnet, dass man den Organismus Streptomyces lydicus ssp. tanzanius NRRL 18036 oder einen Mutanten davon in einem flüssigen Medium, welches assimilierbare Quellen von Kohlenstoff, Stickstoff und anorganischen Salzen enthält, aerob fermentiert bis genanntes Medium eine substantielle antibiotische Aktivität aufweist, und danach das Antibiotikum LL-E19020 Zeta daraus isoliert.

2. Verfahren zur Herstellung des Antibiotikums LL-E19020 Zeta, welches
(a) die Struktur 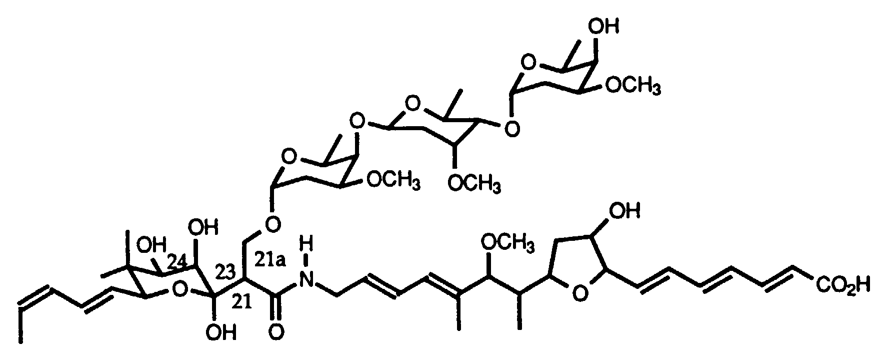
(b) ein Molekulargewicht von 1107 (FABMS=M/Z 1130 entsprechend [M+Na]+);
(c) ein charakteristisches Ultraviolett-Absorptionsspektrum wie in Figur I der beiliegenden Zeichnungen;
(d) ein charakteristisches Infrarot-Absorptionsspektrum wie in Figur II der beiliegenden Zeichnungen;
(e) ein charakteristisches Protonen-NMR-Spektrum wie in Figur III der beiliegenden Zeichnungen;
(f) ein charakteristisches Kohlenstoff-13-NMR-Spektrum wie in Figur IV der beiliegenden Zeichnungen;
(g) eine charakteristische HPLC-Retentionszeit von 13.2 Minuten unter Verwendung eines Eluierungsmittelgradienten von Acetonitril in wässriger Essigsäure; und
(h) eine charakteristische HPLC-Retentionszeit von 16.3 Minuten unter Verwendung eines Eluierungsmittelgradienten von Dioxan in wässriger Essigsäure aufweist,
dadurch gekennzeichnet, dass man ein flüssiges Medium, welches assimilierbare Quellen von Kohlenstoff, Stickstoff und anorganischen Salzen enthält und welches mit einer lebensfähigen Kultur des Organismus Streptomyces lydicus ssp. tanzanius NRRL 18036 oder eines Mutanten davon inokuliert wurde, aerob fermentiert, wobei die Fermentationskultur während etwa 80-200 Stunden bei einer Temperatur von 25-32°C gehalten wird, die Maische abtrennt und das Antibiotikum extrahiert.

3. Verwendung des gemäss Anspruch 1 oder 2 erhältlichen Antibiotikums LL-E19020 Zeta zur Herstellung eines Medikamentes zur Behandlung bakterieller Infektionen in Warmblütern.

4. Verfahren zur Herstellung einer antibiotischen, pharmazeutischen Komposition, dadurch gekennzeichnet, dass man eine antibiotische Menge des gemäss Anspruch 1 oder 2 erhältlichen LL-E19020 Zeta und einen pharmazeutisch verwendbaren Träger formuliert.

5. Verfahren zur Herstellung des Antibiotikums LL-E19020 Eta, welches
(a) die Struktur 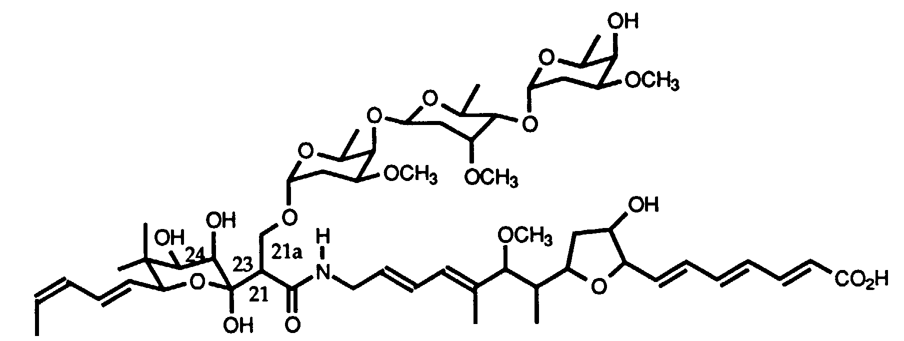
(b) ein Molekulargewicht von 963 (FABMS=M/Z 986 entsprechend [M+Na]+);
(c) ein charakteristisches Ultraviolett-Absorptionsspektrum wie in Figur V der beiliegenden Zeichnungen;
(d) ein charakteristisches Infrarot-Absorptionsspektrum wie in Figur VI der beiliegenden Zeichnungen;
(e) ein charakteristisches Protonen-NMR-Spektrum wie in Figur VII der beiliegenden Zeichnungen;
(f) ein charakteristisches Kohlenstoff-13-NMR-Spektrum wie in Figur VIII der beiliegenden Zeichnungen;
(g) eine charakteristische HPLC-Retentionszeit von 11.5 Minuten unter Verwendung eines Eluierungsmittelgradienten von Acetonitril in wässriger Essigsäure; und
(h) eine charakteristische HPLC-Retentionszeit von 14.2 Minuten unter Verwendung eines Eluierungsmittelgradienten von Dioxan in wässriger Essigsäure aufweist,
dadurch gekennzeichnet, dass man den Organismus Streptomyces lydicus ssp. tanzanius NRRL 18036 oder einen Mutanten davon in einem flüssigen Medium, welches assimilierbare Quellen von Kohlenstoff, Stickstoff und anorganischen Salzen enthält, aerob fermentiert bis genanntes Medium eine substantielle antibiotische Aktivität aufweist, und danach das Antibiotikum LL-E19020 Eta daraus isoliert.

6. Verfahren zur Herstellung des Antibiotikums LL-E19020 Eta, welches
(a) die Struktur 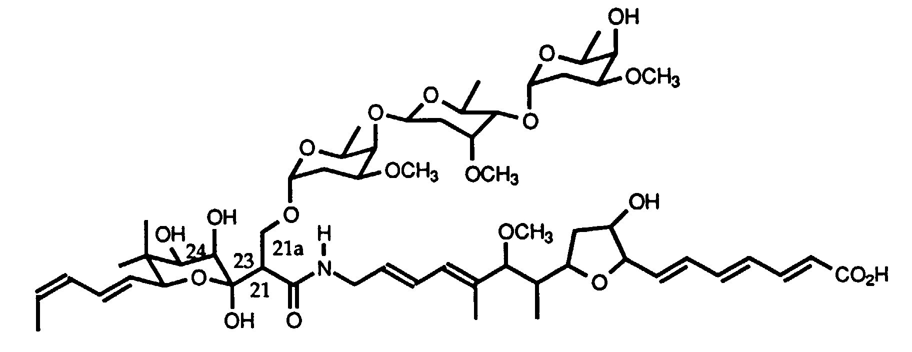
(b) ein Molekulargewicht von 963 (FABMS=M/Z 986 entsprechend [M+Na]+);
(c) ein charakteristisches Ultraviolett-Absorptionsspektrum wie in Figur V der beiliegenden Zeichnungen;
(d) ein charakteristisches Infrarot-Absorptionsspektrum wie in Figur VI der beiliegenden Zeichnungen;
(e) ein charakteristisches Protonen-NMR-Spektrum wie in Figur VII der beiliegenden Zeichnungen;
(f) ein charakteristisches Kohlenstoff-13-NMR-Spektrum wie in Figur VIII der beiliegenden Zeichnungen;
(g) eine charakteristische HPLC-Retentionszeit von 11.5 Minuten unter Verwendung eines Eluierungsmittelgradienten von Acetonitril in wässriger Essigsäure; und
(h) eine charakteristische HPLC-Retentionszeit von 14.2 Minuten unter Verwendung eines Eluierungsmittelgradienten von Dioxan in wässriger Essigsäure aufweist,
dadurch gekennzeichnet, dass man ein flüssiges Medium, welches assimilierbare Quellen von Kohlenstoff, Stickstoff und anorganischen Salzen enthält und welches mit einer lebensfähigen Kultur des Organismus Streptomyces lydicus ssp. tanzanius NRRL 18036 oder eines Mutanten davon inokuliert wurde, aerob fermentiert, wobei die Fermentationskultur während etwa 80-200 Stunden bei einer Temperatur von 25-32°C gehalten wird, die Maische abtrennt und das Antibiotikum extrahiert.

7. Verwendung des gemäss Anspruch 5 oder 6 erhältlichen Antibiotikums LL-E19020 Eta zur Herstellung eines Medikamentes zur Behandlung bakterieller Infektionen in Warmblütern.

8. Verfahren zur Herstellung einer antibiotischen, pharmazeutischen Komposition, dadurch gekennzeichnet, dass man eine antibiotische Menge des gemäss Anspruch 5 oder 6 erhältlichen LL-E19020 Eta und einen pharmazeutisch verwendbaren Träger formuliert.

## Revendications (Revendications pour l'(les) Etat(s) contractant(s) suivant(s): AT, BE, CH, LI, DE, DK, FR, GB, IT, LU, NL, PT, SE)

1. Composé LL-E19020 zêta présentant
(a) la structure suivante 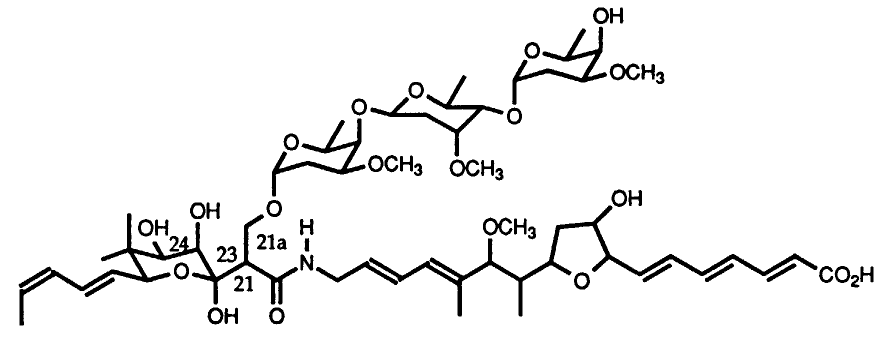
(b) une masse moléculaire de 1 107 (SM-FAB: m/z = 1 130 correspondant à [M+Na]⁺) ;
(c) un spectre caractéristique d'absorption dans l'ultraviolet tel que représenté sur la figure I des dessins ci-annexés;
(d) un spectre caractéristique d'absorption dans l'infrarouge tel que représenté sur la figure II des dessins ci-annexés;
(e) un spectre caractéristique de résonance magnétique nucléaire ¹H tel que représenté sur la figure III des dessins ci-annexés;
(f) un spectre caractéristique de résonance magnétique nucléaire ¹³C tel que représenté sur la figure IV des dessins ci-annexés;
(g) un temps caractéristique de rétention en HPLC de 13,2 minutes, avec utilisation d'un gradient d'acétonitrile dans de l'acide acétique aqueux; et
(h) un temps caractéristique de rétention en HPLC de 16,3 minutes, avec utilisation d'un gradient de dioxanne dans de l'acide acétique aqueux.

2. Procédé pour la production de l'antibiotique LL-E19020 zêta tel que défini dans la revendication 1, comprenant la fermentation aérobie de l'organisme *Streptomyces lydicus* ssp. *tanzanius* NRRL 18036 ou de mutants de celui-ci, dans un milieu liquide contenant des sources assimilables de carbone, d'azote et de sels minéraux, jusqu'à ce qu'une substantielle activité antibiotique soit conférée audit milieu, et ensuite la récupération de l'antibiotique LL-E19020 zêta à partir de ce dernier.

3. Procédé pour la production de l'antibiotique LL-E19020 zêta tel que défini dans la revendication 1, comprenant la fermentation aérobie d'un milieu liquide contenant des sources assimilables de carbone, d'azote et de sels minéraux, lequel milieu a été ensemencé avec une culture viable de l'organisme *Streptomyces lydicus* ssp. *tanzanius* NRRL 18036 ou de mutants de celui-ci, le maintien de ladite culture par fermentation à une température de 25-32°C pendant une durée d'environ 80-200 heures, la récolte du bouillon et l'extraction de l'antibiotique.

4. Utilisation de l'antibiotique LL-E19020 zêta tel que défini dans la revendication 1, pour la fabrication d'un médicament destiné au traitement d'infections bactériennes chez des animaux à sang chaud.

5. Composition pharmaceutique antibiotique comprenant une quantité antibiotique de LL-E19020 zêta tel que défini dans la revendication 1, en association avec un véhicule pharmaceutiquement acceptable.

6. Composé LL-E19020 êta présentant
(a) la structure suivante 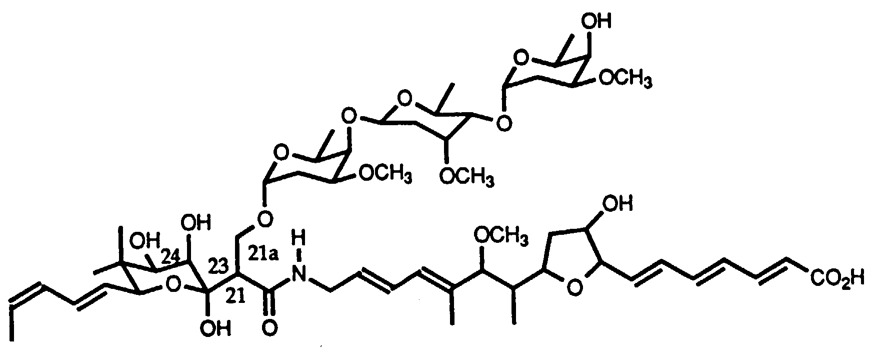
(b) une masse moléculaire de 963 (SM-FAB = m/z 986 correspondant à [M+Na] ⁺) ;
(c) un spectre caractéristique d'absorption dans l'ultraviolet tel que représenté sur la figure V des dessins ci-annexés;
(d) un spectre caractéristique d'absorption dans l'infrarouge tel que représenté sur la figure VI des dessins ci-annexés;
(e) un spectre caractéristique de résonance magnétique nucléaire ¹H tel que représenté sur la figure VII des dessins ci-annexés;
(f) un spectre caractéristique de résonance magnétique nucléaire ¹³C tel que représenté sur la figure VIII des dessins ci-annexés;
(g) un temps caractéristique de rétention en HPLC de 11,59 minutes, avec utilisation d'un gradient d'acétonitrile dans de l'acide acétique aqueux; et
(h) un temps caractéristique de rétention en HPLC de 14,2 minutes, avec utilisation d'un gradient de dioxanne dans de l'acide acétique aqueux.

7. Procédé pour la production de l'antibiotique LL-E19020 êta tel que défini dans la revendication 6, comprenant la fermentation aérobie de l'organisme *Streptomyces lydicus* ssp. *tanzanius* NRRL 18036 ou de mutants de celui-ci, dans un milieu liquide contenant des sources assimilables de carbone, d'azote et de sels minéraux, jusqu'à ce qu'une substantielle activité antibiotique soit conférée audit milieu, et ensuite la récupération de l'antibiotique LL-E19020 êta à partir de ce dernier.

8. Procédé pour la production de l'antibiotique LL-E19020 êta tel que défini dans la revendication 6, comprenant la fermentation aérobie d'un milieu liquide contenant des sources assimilables de carbone, d'azote et de sels minéraux, lequel milieu a été ensemencé avec une culture viable de l'organisme *Streptomyces lydicus* ssp. *tanzanius* NRRL 18036 ou de mutants de celui-ci, le maintien de ladite culture par fermentation à une température de 25-32°C pendant une durée d'environ 80-200 heures, la récolte du bouillon et l'extraction de l'antibiotique.

9. Utilisation de l'antibiotique LL-E19020 êta tel que défini dans la revendication 6, pour la fabrication d'un médicament destiné au traitement d'infections bactériennes chez des animaux à sang chaud.

10. Composition pharmaceutique antibiotique comprenant une quantité antibiotique de LL-E19020 êta, tel que défini dans la revendication 6, en association avec un véhicule pharmaceutiquement acceptable.

## Revendications (Revendications pour l'(les) Etat(s) contractant(s) suivant(s): ES, GR)

1. Procédé de production de l'antibiotique LL-E19020 zêta présentant
(a) la structure suivante 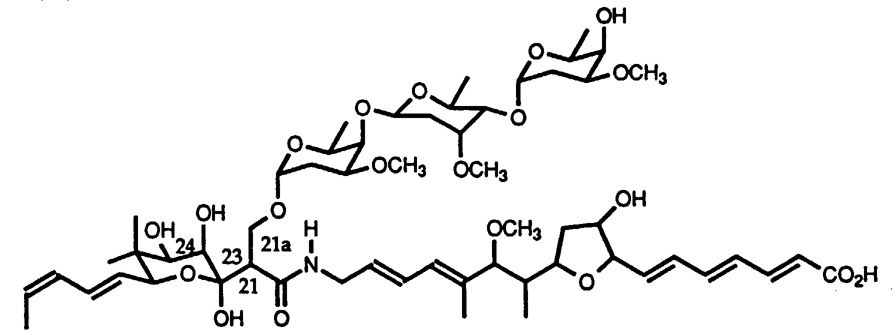
(b) une masse moléculaire de 1 107 (SM-FAB: m/z = 1 130 correspondant à [M+Na]⁺) ;
(c) un spectre caractéristique d'absorption dans l'ultraviolet tel que représenté sur la figure I des dessins ci-annexés;
(d) un spectre caractéristique d'absorption dans l'infrarouge tel que représenté sur la figure II des dessins ci-annexés;
(e) un spectre caractéristique de résonance magnétique nucléaire ¹H tel que représenté sur la figure III des dessins ci-annexés;
(f) un spectre caractéristique de résonance magnétique nucléaire ¹³C tel que représenté sur la figure IV des dessins ci-annexés;
(g) un temps caractéristique de rétention en HPLC de 13,2 minutes, avec utilisation d'un gradient d'acétonitrile dans de l'acide acétique aqueux; et
(h) un temps caractéristique de rétention en HPLC de 16,3 minutes, avec utilisation d'un gradient de dioxanne dans de l'acide acétique aqueux,
comprenant la fermentation aérobie de l'organisme *Streptomyces lydicus* ssp. *tanzanius* NRRL 18036 ou de mutants de celui-ci, dans un milieu liquide contenant des sources assimilables de carbone, d'azote et de sels minéraux, jusqu'à ce qu'une substantielle activité antibiotique soit conférée audit milieu, et ensuite la récupération de l'antibiotique LL-E19020 zêta à partir de ce dernier.

2. Procédé de production de l'antibiotique LL-E19020 zêta présentant
(a) la structure suivante 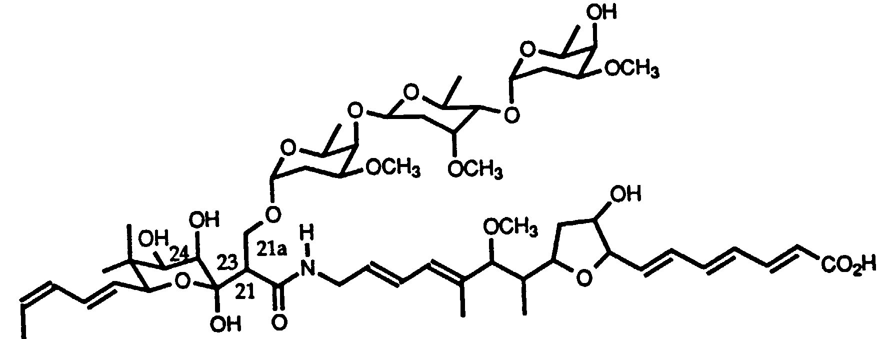
(b) une masse moléculaire de 1 107 (SM-FAB: m/z = 1 130 correspondant à [M+Na]⁺) ;
(c) un spectre caractéristique d'absorption dans l'ultraviolet tel que représenté sur la figure I des dessins ci-annexés;
(d) un spectre caractéristique d'absorption dans l'infrarouge tel que représenté sur la figure II des dessins ci-annexés;
(e) un spectre caractéristique de résonance magnétique nucléaire ¹H tel que représenté sur la figure III des dessins ci-annexés;
(f) un spectre caractéristique de résonance magnétique nucléaire ¹³C tel que représenté sur la figure IV des dessins ci-annexés;
(g) un temps caractéristique de rétention en HPLC de 13,2 minutes, avec utilisation d'un gradient d'acétonitrile dans de l'acide acétique aqueux; et
(h) un temps caractéristique de rétention en HPLC de 16,3 minutes, avec utilisation d'un gradient de dioxanne dans de l'acide acétique aqueux,
comprenant la fermentation aérobie d'un milieu liquide contenant des sources assimilables de carbone, d'azote et de sels minéraux, lequel milieu ayant été ensemencé avec une culture viable de l'organisme *Streptomyces lydicus* ssp. *tanzanius* NRRL 18036 ou de mutants de celui-ci, le maintien de ladite culture par fermentation à une température de 25-32°C pendant une durée d'environ 80-200 heures, la récolte du bouillon et l'extraction de l'antibiotique.

3. Utilisation de l'antibiotique LL-E19020 zêta pouvant être obtenu selon la revendication 1 ou 2, pour la fabrication d'un médicament destiné au traitement d'infections bactériennes chez des animaux à sang chaud.

4. Procédé pour la préparation d'une composition pharmaceutique antibiotique, comprenant la formulation d'une quantité antibiotique de LL-E19020 zêta pouvant être obtenu selon la revendication 1 ou 2, avec un véhicule pharmaceutiquement acceptable.

5. Procédé de production de l'antibiotique LL-E19020 êta présentant
(a) la structure suivante 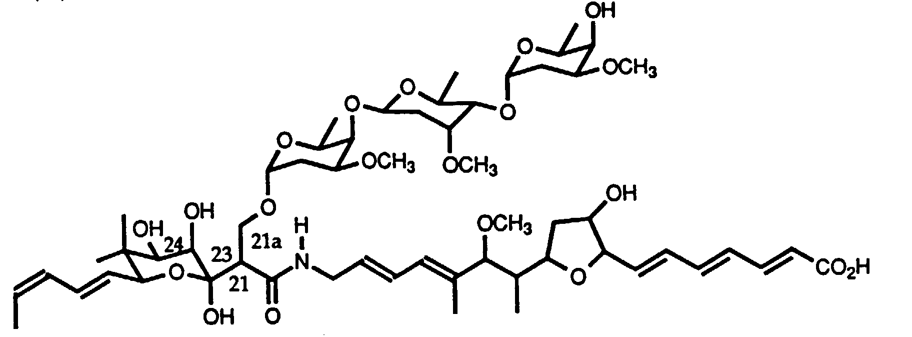
(b) une masse moléculaire de 963 (SM-FAB = m/z 986 correspondant à [M+Na]⁺);
(c) un spectre caractéristique d'absorption dans l'ultraviolet tel que représenté sur la figure V des dessins ci-annexés;
(d) un spectre caractéristique d'absorption dans l'infrarouge tel que représenté sur la figure VI des dessins ci-annexés;
(e) un spectre caractéristique de résonance magnétique nucléaire ¹H tel que représenté sur la figure VII des dessins ci-annexés;
(f) un spectre caractéristique de résonance magnétique nucléaire ¹³C tel que représenté sur la figure VIII des dessins ci-annexés;
(g) un temps caractéristique de rétention en HPLC de 11,59 minutes, avec utilisation d'un gradient d'acétonitrile dans de l'acide acétique aqueux; et
(h) un temps caractéristique de rétention en HPLC de 14,2 minutes, avec utilisation d'un gradient de dioxanne dans de l'acide acétique aqueux,
comprenant la fermentation aérobie de l'organisme *Streptomyces lydicus* ssp. *tanzanius* NRRL 18036 ou de mutants de celui-ci, dans un milieu liquide contenant des sources assimilables de carbone, d'azote et de sels minéraux, jusqu'à ce qu'une substantielle activité antibiotique soit conférée audit milieu, et ensuite la récupération de l'antibiotique LL-E19020 êta à partir de ce dernier.

6. Procédé de production de l'antibiotique LL-E19020 êta présentant
(a) la structure suivante 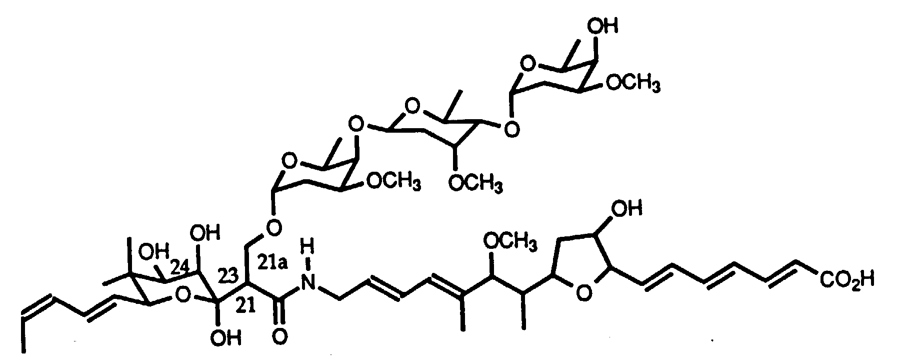
(b) une masse moléculaire de 963 (SM-FAB = m/z 986 correspondant à [M+Na]⁺) ;
(c) un spectre caractéristique d'absorption dans l'ultraviolet tel que représenté sur la figure V des dessins ci-annexés;
(d) un spectre caractéristique d'absorption dans l'infrarouge tel que représenté sur la figure VI des dessins ci-annexés;
(e) un spectre caractéristique de résonance magnétique nucléaire ¹H tel que représenté sur la figure VII des dessins ci-annexés;
(f) un spectre caractéristique de résonance magnétique nucléaire ¹³C tel que représenté sur la figure VIII des dessins ci-annexés;
(g) un temps caractéristique de rétention en HPLC de 11,59 minutes, avec utilisation d'un gradient d'acétonitrile dans de l'acide acétique aqueux; et
(h) un temps caractéristique de rétention en HPLC de 14,2 minutes, avec utilisation d'un gradient de dioxanne dans de l'acide acétique aqueux,
comprenant la fermentation aérobie d'un milieu liquide contenant des sources assimilables de carbone, d'azote et de sels minéraux, lequel milieu ayant été ensemencé avec une culture viable de l'organisme *Streptomyces lydicus* ssp. *tanzanius* NRRL 18036 ou de mutants de celui-ci, le maintien de ladite culture par fermentation à une température de 25-32°C pendant une durée d'environ 80-200 heures, la récolte du bouillon et l'extraction de l'antibiotique.

7. Utilisation de l'antibiotique LL-E19020 êta pouvant être obtenu selon la revendication 5 ou 6, pour la fabrication d'un médicament destiné au traitement d'infections bactériennes chez des animaux à sang chaud.

8. Procédé pour la préparation d'une composition pharmaceutique antibiotique, comprenant la formulation d'une quantité antibiotique de LL-E19020 êta, pouvant être obtenu selon la revendication 5 ou 6, avec un véhicule pharmaceutiquement acceptable.
